# EUROPEAN PATENT SPECIFICATION

(11) **EP 3 014 274 B1**
(45) Date of publication and mention of the grant of the patent: **09.08.2017**
(21) Application number: 14738423.4
(22) Date of filing: 23.06.2014
(51) Int. Cl.: G01N 33/542, G01N 33/58, C12Q 1/34, C09K 11/02, C09K 11/88

(54) **RATIOMETRIC ASSAY FOR HYDROLYTIC ENZYME QUANTIFICATION**
RATIOMETRISCHER TEST ZUR QUANTIFIZIERUNG VON HYDROLYTISCHEN ENZYMEN
DOSAGE RATIOMÉTRIQUE POUR QUANTIFICATION D'ENZYMES HYDROLYTIQUES

(30) Priority: 25.06.2013 EP 13382239
(43) Date of publication of application: 04.05.2016
(73) Proprietor: Fundació Institut Català d'Investigació Química, 43007 Tarragona (ES); Institució Catalana de Recerca i Estudis Avançats, 08010 Barcelona (ES)
(72) Inventor: PALOMARES, Emilio, E-43007 Tarragona (ES); STOICA, Georgiana, E-43006 Tarragona (ES); CASTELLÓ SERRANO, Iván, E-46400 Cullera (ES)
(74) Representative: ZBM Patents - Zea, Barlocci & Markvardsen
(86) International application number: PCT/EP2014/063117
(87) International publication number: WO 2014/206918

(56) References cited:
- QIANG MA ET AL: "Multilayered, core/shell nanoprobes based on magnetic ferric oxide particles and quantum dots for multimodality imaging of breast cancer tumors", BIOMATERIALS, vol. 33, no. 33, 17 August 2012 (2012-08-17), pages 8486-8494, XP055082982,
- SAPSFORD K E ET AL: "Monitoring of enzymatic proteolysis on a electroluminescent-CCD microchip platform using quantum dot-peptide substrates", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 139, no. 1, 20 May 2009 (2009-05-20), pages 13-21, XP026095280, ISSN: 0925-4005, DOI: 10.1016/J.SNB.2008.07.026 [retrieved on 2008-08-05] cited in the application
- YOSHIO KOBAYASHI ET AL: "Control of shell thickness in silica-coating of Au nanoparticles and their X-ray imaging properties", JOURNAL OF COLLOID AND INTERFACE SCIENCE, ACADEMIC PRESS, NEW YORK, NY, US, vol. 358, no. 2, 14 January 2011 (2011-01-14), pages 329-333, XP028197832, ISSN: 0021-9797, DOI: 10.1016/J.JCIS.2011.01.058 [retrieved on 2011-01-22]

## Description

The present invention relates to the field of analytical biochemistry, particularly to the determination of biological compounds by means of ratiometric sensing. The present invention is useful, for example, in the field of clinical diagnosis, particularly in the diagnosis of cystic fibrosis.

### BACKGROUND ART

The field of clinical diagnosis is in constant search for new methods to detect key biomolecules that constitute relevant biomarkers for a wide range of diseases. In addition to high standards of specificity and sensitivity, this field requires that the tests are rapid, easy to use and may be performed using standard instrumentation. Further, clinical tests are preferred that are user-friendly and suitable to apply at the point of care, for which they need to be scaled down to small devices.

Hydrolytic enzymes play key functions in many normal biological processes. As such, the monitoring of hydrolytic enzymes is of outmost importance to the field of clinical diagnosis, as well as to other fields such as food industry and biopharmaceutical industry. Over the past decade, probing of hydrolytic enzymes has mostly been relying on affinity-based methods such as immunoassays, rather than the activity-based assays which can provide higher specificity. Also, while requiring several reaction steps and different reagents, immunoassays such as ELISA are usually time-consuming and not suitable for point of care application. Furthermore, immunoassays sometimes lack specificity due to cross-reactivity. Other approaches to assay hydrolytic activity have been reported based on liquid chromatography or gel electrophoresis, but most of them are time-consuming and laborious.

An example of a disease-relevant hydrolytic enzyme is trypsin. Trypsin is relevant in cystic fibrosis disease (from now on CF), an autosomal recessive genetic disorder that affects most critically the pancreas, the lungs, and also the liver and intestine. CF is caused by a mutation in the gene for the protein cystic fibrosis transmembrane conductance regulator (CFTR). Babies with cystic fibrosis may suffer a build-up of trypsinogen (the trypsin precursor) in the blood because mucus blocks the passageways between the pancreas and the small intestine, meaning that trypsin cannot reach the intestine. Most countries have established a default screening for newborns that involves quantifying trypsinogen in the blood of the babies (immunoreactive trypsinogen test, or IRT), which typically makes use of fluorescent-based immunoassays, such as DELFIA Neonatal IRT kits (PerkinElmer). However, these tests still have the drawbacks of immunoassays with respect to specificity, time-effectiveness and user-friendliness. Additionally, due to a considerable percentage of false positives inherent to this test, babies with abnormal results must be either tested again for IRT or, alternatively, undergo genetic testing to determine whether they have at least one mutated copy of the CFTR gene (carriers). Infants with a second abnormal newborn IRT screen or carrier genotype need a sweat test to confirm the CF diagnosis. This procedure is long and sometimes considered invasive by the parents.

A different approach for the monitoring of trypsin enzymatic activity has been described by Sapsford *et al.* These authors described an electroluminescent assay using quantum dots (QD)-peptide substrates that can be cleaved specifically by the trypsin enzyme ("Monitoring of enzymatic proteolysis on a electroluminescent-CCD microchip platform using quantum dot-peptide substrates", Sensors and Actuators B, 2009, vol. 139, pp. 13-21). Said system takes advantage of Förster fluorescence resonance energy transfer (FRET) between the quantum dot and a label born by the peptide substrate. By action of the enzyme, the variation in the optical response of the system can be measured to provide a qualitative determination of trypsin activity. This system, however, is not able to provide an accurate quantification of trypsin activity.

Qiang Ma et al, ("Multilayered, core/shell nanoprobes based on magnetic ferric oxide particles and quantum dots for multimodality imaging of breast cancer tumors", Biomaterials, 2012, vol. 33, pp. 8486-8494) disclose bioconjugated, multi silica-layered core/shell nanoprobes based on magnetic ferric oxide particles and quantum dots used for diagnostic purposes, in particular for diagnosing breast cancer. The fabrication of the disclosed nanoprobes is performed by a reverse microemulsion method.

It is therefore desirable to provide alternative bioassays to accurately quantify hydrolytic enzyme activity, particularly trypsin activity, which do not have the drawbacks mentioned above.

### SUMMARY OF THE INVENTION

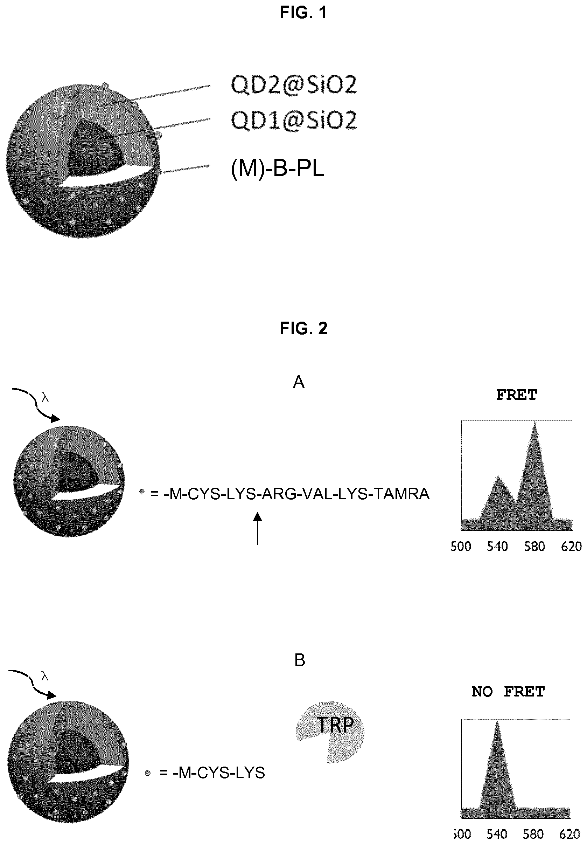

The inventors have developed a high-performance ratiometric bioassay for the quantification of hydrolytic enzyme activity. Said assay takes advantage of the FRET process that occurs within specifically designed nanoparticles comprising a first population of QDs (QD1), a second population of QDs (QD2), a biomolecule that comprises a cleavage site susceptible of being cleaved by a hydrolytic enzyme and a photoluminescent label. A schematic representation of the nanoparticle of the invention can be seen in FIG 1.

Thus a first aspect of the invention refers to a nanoparticle comprising (i) a core comprising a first population of quantum dots (QDs) embedded in silica, (ii) a shell comprising a second population of QDs embedded in silica, (iii) at least one biomolecule selected from a peptide, a nucleic acid, a carbohydrate or a lipid which comprises a cleavage site that is susceptible of being cleaved by a hydrolytic enzyme, said biomolecule being bound to the surface of the shell through a moiety selected from the moieties of formula (I) and (II), 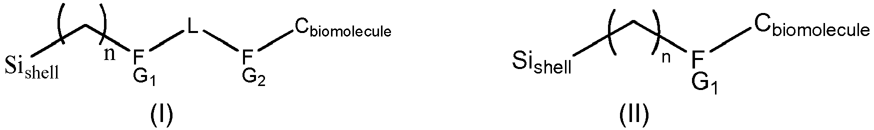 wherein Siₛₕₑₗₗ is a silicium atom comprised in the shell and C_{biomolecule} is a carbon atom comprised in the biomolecule; n is an integer comprised of from 0 to 10; FG₁ and FG₂ are each a diradical independently selected from the group consisting of the diradicals of formula (III), (IV), (V), (VI), (VII), (VIII) and (IX) 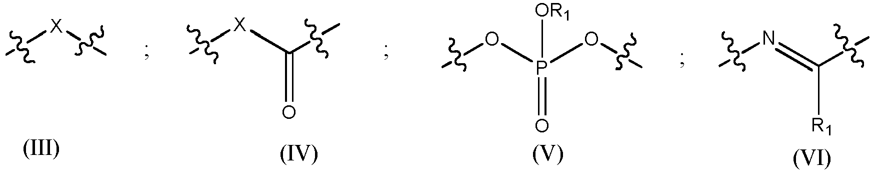 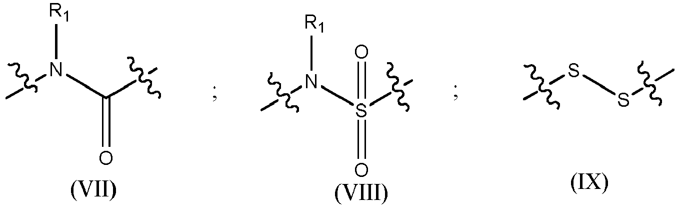 where X is S or O; R₁ is H or (C₁-C₁₂)alkyl; L is a diradical selected from the diradicals deriving from a compound selected from (C₁-C₁₂)alkane, (C₁-C₁₂)alkene, (C₁-C₁₂)alkyne, (C₃-C₆)cycloalkane and benzene, and (iv) a photoluminescent label for each biomolecule, wherein the label is bound to the part of the biomolecule which detaches from the nanoparticle after cleavage of said biomolecule by a hydrolytic enzyme through a diradical of formula FG₃ which is selected from the group consisting of the diradicals of formula (III), (IV), (V), (VI), (VII), (VIII) and (IX) defined above; wherein the first and second QD populations have different maximum photoluminescence emission wavelengths, and only the second QD population is susceptible of producing Förster resonance energy transfer (FRET) with the photoluminescent label.

The nanoparticles of the invention can be used in bioassays; particularly, they can be used for quantifying of a hydrolytic enzyme in a sample. Thus, another aspect of the invention refers to the use of the nanoparticle of the invention for quantifying of a hydrolytic enzyme in a sample. The specific design of the nanoparticles provides high sensitivity, repeatability and accuracy in hydrolytic enzyme quantification. When the nanoparticles comprise a peptide containing a site susceptible of being cleaved by trypsin, said nanoparticles can be used for the diagnosis of cystic fibrosis. Thus, another aspect of the invention refers to a nanoparticle of the invention, wherein the nanoparticle comprises a peptide comprising a cleavage site susceptible of being cleaved by trypsin, as a diagnostic agent for cystic fibrosis.

The present invention also refers to a method for quantifying a hydrolytic enzyme in a sample comprising the steps of: (i) contacting a nanoparticle as described above with the sample, and (ii) determining the photoluminescent (PL) intensity of the QD1 and QD2 upon exposing the mixture of step (i) to an excitation light source capable of exciting the QD1 and QD2, to emit a PL emission, wherein the relation between the photoluminescent intensities of QD2 and QD1 is correlated with the quantity of hydrolytic enzyme in the sample. A schematic representation of a particular embodiment of the method of the invention is shown in FIG 2.

The present assay thus provides sensitive ratiometric quantification of hydrolytic enzymes, which renders said method clearly superior to prior photoluminescent assays. In contrast to other conventional assays for enzyme quantification, such as immunoassays, the present invention provides time-effective quantification results with minimum manipulation (one-step method), and cross-reactivity is avoided thanks to the high specificity of the enzyme cleavage site. Moreover, the bioassay of the invention may be downscaled into a small device to be used in point-of-care diagnosis and, thanks to its robustness, it can be directly employed to analyse biological samples. These advantages render the bioassay of the invention particularly appropriate for diagnosis purposes. When applied to CF diagnosis, the method is non-invasive as well as reliable, overcoming most of the drawbacks of conventional CF diagnosis methods.

Another aspect of the invention refers to a method for the diagnosis of cystic fibrosis comprising the steps of: (i) contacting a faeces sample obtained from a patient with a nanoparticle as defined above which comprises a peptide comprising a site susceptible of being cleaved by trypsin, and (ii) determining the PL intensity of the QD1 and QD2 upon exposing the mixture of step (i) to an excitation light source capable of exciting the QD1 and QD2, to emit a PL emission, wherein the relation between the PL intensities of QD2 and QD1 is correlated with the quantity of trypsin in the faeces, and wherein a trypsin concentration comprised from 0 to 90 µg/g is indicative of cystic fibrosis.

Finally, another aspect of the invention refers to a method for preparing a nanoparticle as defined above. Said method comprises (i) forming a core comprising a first population of silica embedded QDs by the reverse microemulsion method, (ii) forming a shell containing a second population of silica embedded QDs by subjecting the core comprising a first population of silica embedded QDs of step (i) to reverse microemulsion method, (iii) recovering the core-shell nanoparticle obtained in step (ii), and optionally functionalising the surface of the shell, and (iv) adding at least one biomolecule selected from a peptide, a nucleic acid, a carbohydrate or a lipid, wherein each biomolecule comprises a cleavage site that is susceptible of being cleaved by a hydrolytic enzyme, and a photoluminescent label that is bound to the part of the biomolecule which detaches from the nanoparticle after cleavage of said biomolecule by a hydrolytic enzyme through a diradical of formula FG₃ as previously defined; whereby the biomolecule is bound to the surface of the shell through a moiety selected from the moieties of formula (I) and (II) as previously defined.

### BRIEF DESCRIPTION OF THE DRAWINGS

FIG. 1. Schematic representation of a nanoparticle of the invention. The nanoparticle comprises a core of silica-embedded first QD population (QD1@SiO2), a shell of a second silica-embedded QD population (QD2@SiO2), and a photoluminescent-labelled (PL) biomolecule (B) which is bound to the surface of the shell either directly or through a moiety (M).
FIG. 2. Schematic representation of trypsin quantification by the method of the invention using a nanoparticle that comprises a TAMRA-labelled peptide of SEQ ID NO: 2 with cleavage site for trypsin (the cleavage site is indicated by an arrow) which is bound to the surface of the shell by a moiety M. A, in the absence of trypsin, FRET is observed within the nanoparticle. B, in the presence of trypsin, the peptide is digested and FRET is interrupted.

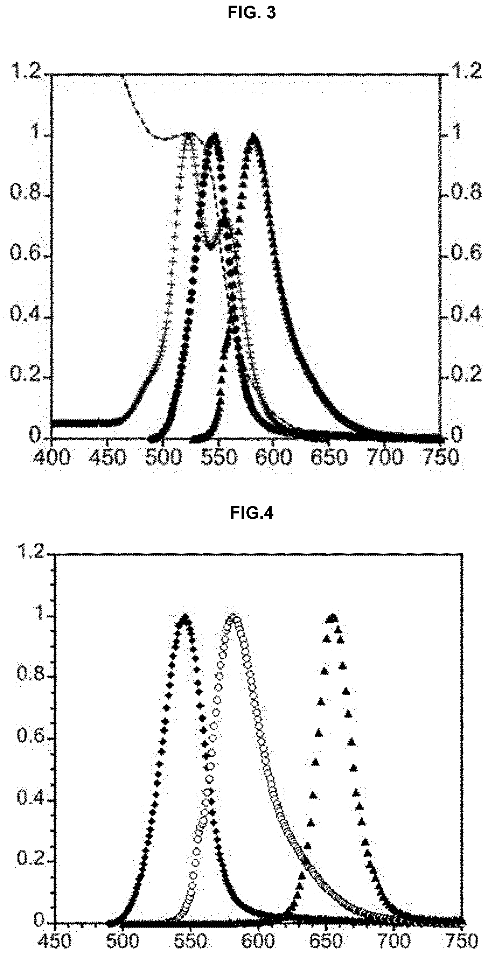

FIG. 3. Normalized absorption spectra of QD540@SiO2 nanospheres (dash line) and TAMRA (+ symbol) and normalized emission spectra of QD540@SiO2 nanospheres (circle) and TAMRA (triangle). X axis represents the wavelength, expressed in nm.
FIG. 4. From left to right: emission spectra of QD540, TAMRA and QD660. X axis represents wavelength (nm). Y axis represents normalized fluorescence intensity in fluorescent units (FU).

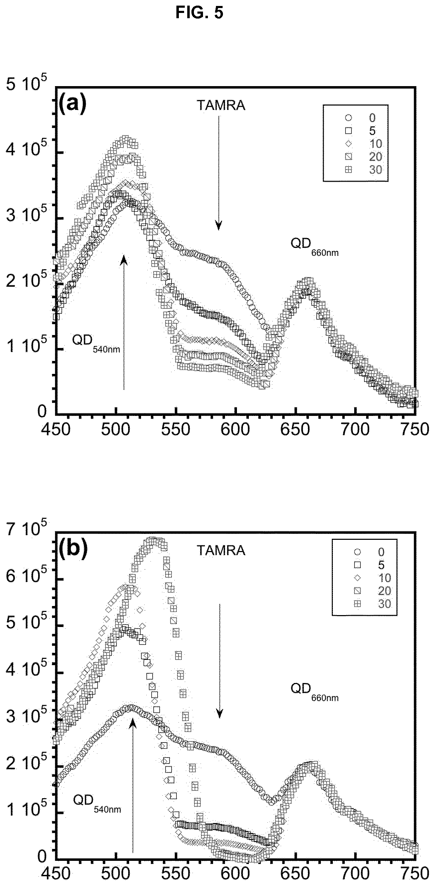

FIG. 5. QD660@SiO2@QD540@SiO2 emission spectra (a) after digestion with 25 µ/L of trypsin, and (b) after digestion with 350 µg/L trypsin. The figure legends correspond to the enzymatic digestion time in minutes (0-30 min). X axis represents wavelength (nm). Y axis represents PL intensity in fluorescent units (FU).

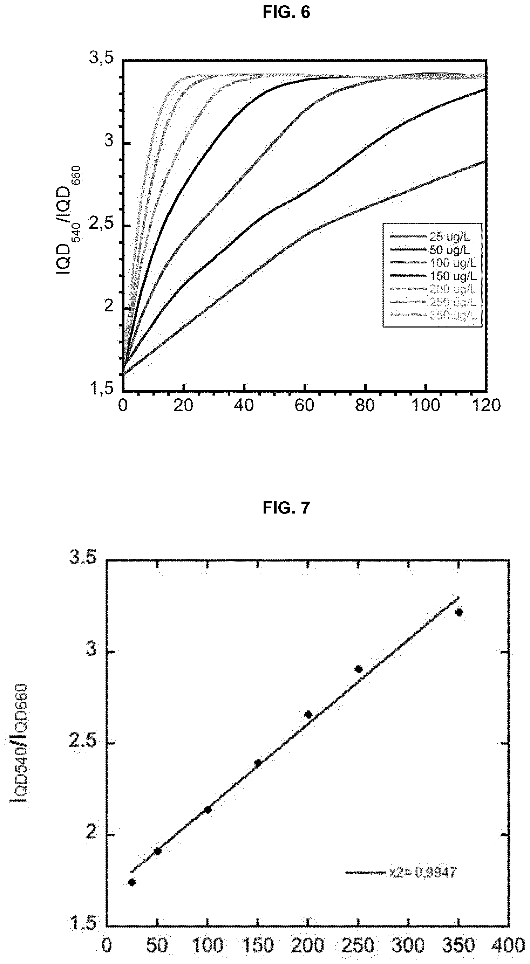

FIG. 6. Kinetics of the enzymatic digestion as a function of trypsin concentration using the ratio between PL emission peaks of both QD540 and QD660 at the QD660@SiO2@QD540@SiO2 nanoparticles. Curves from left to right: 350, 250, 200, 150, 100, 50 and 25 µg/L of trypsin concentration. X axis represents time (min).
FIG. 7. Calibration curve resulting from fitting the fluorescence I₅₄₀/I₆₆₀ experimental values from FIG. 6 at the enzyme digestion time of 10 minutes. X axis represents trypsin concentration (µg/L).

### DETAILED DESCRIPTION OF THE INVENTION

The term "photoluminescence" (abbreviated as PL) refers to a process in which a molecule, in this case the QD, absorbs photons (electromagnetic radiation) and then re-radiates photons. Photoluminescence includes fluorescence and phosphorescence. In the case of fluorescence, upon absorption of electromagnetic radiation by the molecule (in the case of the invention, QD or label), an electron of the Highest Occupied Molecular Orbital (HOMO) jumps to a higher energy level corresponding to the excited state (Lowest Unoccupied Molecular Orbital (LUMO)), the energy gap between HOMO and LUMO corresponding to the energy of the electromagnetic radiation, characterized by its wavelength. The electron occupying the LUMO then relaxes at a lower energy state, which provokes the emission of electromagnetic radiation at a given wavelength, usually different from the absorption wavelength. The period between absorption and emission is typically extremely short, in the order of 10 nanoseconds. In the sense of the invention, PL refers particularly to fluorescence, where some of the original energy is dissipated so that the emitted light photons are of lower energy than those absorbed. The generated photon in this case is said to be red shifted, referring to the loss of energy. Another type of PL which is also contemplated in the present invention is phosphorescence, in which the energy from absorbed photons undergoes intersystem crossing into a state of higher spin multiplicity (see term symbol), usually a triplet state. Once the energy is trapped in the triplet state, transition back to the lower singlet energy states is quantum mechanically forbidden, meaning that it happens much more slowly than other transitions. The result is a slow process of radiative transition back to the singlet state, sometimes lasting minutes or hours. Photoluminescence can be expressed as absorption and emission intensities at given wavelengths. Preferably, the wavelength of emission is higher than the wavelength of absorption.

PL molecules have a range of wavelengths that can excite the molecule to varying extents and, equally, PL molecules can emit light of different wavelengths, these respectively being called absorption and emission spectrum.

The term "photoluminescent label" or "PL label" or "label" refers to a compound that can re-emit light upon light excitation. In the present invention, a PL label must be able to covalently bind to another molecule, particularly, to the part of the biomolecule containing a cleavage site for a hydrolytic enzyme which detaches from the nanoparticle after cleavage of said biomolecule by the hydrolytic enzyme. The PL label contained in the nanoparticle of the invention has emission and absorption spectra which enable the establishment of FRET between the QDs contained in the shell of the nanoparticle (second QD population) and said label. The average distance between said QDs contained in the shell and the PL label is appropriate for the establishment of FRET.

"Förster fluorescence resonance energy transfer" or "FRET" refers to a process based on two photoluminescent substances where initially one of the substances (the donor) can transfer energy to the second one (the acceptor). The efficiency of this energy transfer process is inversely proportional to the sixth power of the distance between donor and acceptor making FRET extremely sensitive to short distances between the two substances. Consequently, FRET is observed when "donor" and "acceptor" are sufficiently close one to another and when there is sufficient spectral overlap between the emission spectrum of the donor and absorption spectrum of acceptor.

The "spectral overlap" is the area of overlap between the donor emission and the acceptor absorption. The spectral overlap at a given wavelength or wavelength range can be calculated as the ratio of the surface area of normalized PL emission intensity, expressed in counts, of the photoluminescent acceptor to the surface area of normalized absorbance, expressed in counts, of the photoluminescent donor. The spectral overlap is expressed as a percentage.

The term "quantum dots" (QDs) is understood in the state of the art as photoluminescent semiconductor nanocrystals whose characteristics are closely related to the size and shape of the individual crystal. A QD is capable of emitting electromagnetic radiation or light upon excitation (i.e., the QD is luminescent). Generally, the smaller the size of the crystal, the larger the band gap, the greater the difference in energy between the highest valence band and the lowest conduction band becomes, therefore more energy is needed to excite the dot, and concurrently, more energy is released when the crystal returns to its resting state. For example, in fluorescent dye applications, this equates to higher frequencies of light emitted after excitation of the dot as the crystal shrinks to smaller sizes, resulting in a color shift from red to blue in the emitted light. Thus a main advantage with QDs is that their photoluminescent properties can be tuned by controlling the growth of the crystal while produced. In addition to such size-tuneable photoluminescence, QDs have broad excitation spectra with narrow emission bandwidths that span the visible spectrum, allowing simultaneous excitation of several particle sizes at a single wavelength. QDs also have exceptional photochemical stability. Altogether, QDs are an advantageous alternative to conventional organic dyes for bioanalytical applications.

A QD is comprised of a first semiconductor material that sometimes can be coated by a second semiconductor material. The coating material will preferably have a bandgap energy that is larger than the bandgap energy of the first material and may be chosen to have an atomic spacing close to that of the first material. The first and/or second semiconductor material can be, but is not limited to, ZnS, ZnSe, ZnTe, US, CdSe, CdTe, HgS, HgSe, HgTe, MgS, MgSe, MgTe, CaS, CaSe, CaTe, SrS, SrSe, SrTe, BaS, BaSe, BaTe, GaN, GaP, GaAs, GaSb, InN, InP, InAs, InSb, PbSe, or an alloy or mixture thereof. In particular embodiments the QDs are constituted by CdSe, CdS, CdSe coated with ZnS (CdSe/ZnS), or by CdS coated with ZnS (CdS/ZnS).

QD size is usually comprised from about 1 nm to about 50 nm in diameter, sometimes from 2 nm to 20 nm, sometimes from 2 nm to 10 nm. QDs may be prepared by methods well established in the state of the art.

As used herein, the term "population" with regard to QDs refers to a plurality of QDs sharing a common wavelength of maximum emission, and therefore a similar particle diameter.

By "silica embedded QDs" (herein sometimes expressed as QD@SiO2) it is understood that the QDs are dispersed inside a silica matrix. The silica embedded QDs are non-homogeneously distributed throughout the silica matrix. This configuration is provided by the preparation method, where the QDs are subjected to a previously described reverse microemulsion method. The configuration of the nanoparticle can be therefore controlled by the preparation method, particularly, by the reaction time and stoichiometry of the reactants. For example, if the QDs are reacted with enough silica by the reverse emulsion method to form the core for a longer time, the result will be a larger core with a configuration where the outer part has a very low, or even non-existant, QD concentration. Alternatively, different proportions of QDs and silica as starting material for the reverse microemulsion method will yield different nanoparticles with regard to their QD-silica configuration.

A "hydrolytic enzyme", also called "hydrolase", is an enzyme that catalyzes the hydrolysis of a chemical bond. Hydrolases are classified as EC 3 in the EC number classification of enzymes but can be further classified into several subclasses, based on the bonds they act upon. Non-limitative examples of hydrolytic enzymes are: proteases (also termed peptidases or proteinases), which cleave peptide bonds; nucleases, which cleave phosphodiester bonds between the nucleotide subunits of nucleic acids; lipases, which catalyse the breakdown of fats by cleaving ester bonds; and glycosidases (also termed glycosyl hydrolases), that catalyze the hydrolysis of the glycosidic linkage in carbohydrates.

The term "cleavage site," refers to the site within a biomolecule, for instance a peptide, in which the backbone of said biomolecule is selectively broken by the hydrolytic enzyme.

The term "functional group" is understood as generally in the state of the art, i.e., as a specific group of atoms or bonds within a molecule that is responsible for the characteristic chemical reactions of that molecule. The same functional group will undergo the same or similar chemical reaction(s) regardless of the size of the molecule it is a part of.

The term "moiety" in the sense of the present invention is understood as a chemical radical that links the biomolecule which comprises a site susceptible of being cleaved by a hydrolytic enzyme to the surface of the shell.

The term "(C₁-C₁₂)-alkyl" as used herein refers to a saturated branched or linear hydrocarbon side chain with 1 to 12 carbon atoms. Preferably "(C₁-C₁₂)-alkyl" is (C₁-C₄)-alkyl, which is preferably an unsubstituted group selected from methyl, ethyl, propyl, isopropyl, butyl, isobutyl, s-butyl and t-butyl. By diradicals deriving from a (C₁-C₁₂)alkane it is understood diradicals constituted by saturated branched or linear hydrocarbon chain with 1 to 12 carbon atoms, for example: -CH₂-CH₂-CH₂- (diradical deriving from propane). Similarly, diradicals deriving from a (C₁-C₁₂)alkene or a (C₁-C₁₂)alkyne are diradicals constituted by unsaturated branched or linear hydrocarbon chains with 1 to 12 carbon atoms, for example: -CH₂-CH=CH- (diradical deriving from propylene) or -CH₂-C≡C- (diradical deriving from propyne). Similarly, diradicals deriving from (C₃-C₆)cycloalkane are diradicals constituted by saturated cyclic hydrocarbon chains with 1 to 12 carbon atoms.

The inventors have developed a method for the quantification of hydrolytic enzyme activity that takes advantage of the FRET process that occurs within newly developed nanoparticles. Said nanoparticles contain two concentrically colour-encoded silica-embedded QD populations and a PL-marked biomolecule which comprises a cleavage site susceptible of being cleaved by a hydrolytic enzyme, said biomolecule being bound to the surface of the shell through a moiety of formula (I) or (II) as defined above (see FIGs 1 and 2). The type of moiety binding the biomolecule to the surface of the shell partly depends on the chemical nature of said biomolecule.

In one embodiment, the biomolecule is bound to the surface of the shell through a moiety of formula (I). In another embodiment, the biomolecule is bound to the surface of the shell through a moiety of formula (II). Said moieties are introduced during the preparation of the nanoparticles in order to provide the surface of the shell with a desired reactivity as explained below.

In one embodiment, in the moiety of formula (I), n is an integer comprised from 1 to 5. In one embodiment, in the moiety of formula (I), L is a diradical selected from the diradicals deriving from (C₁-C₁₂)alkane, (C₃-C₆)cycloalkane and benzene. In a particular embodiment, in the moiety of formula (I), L is a diradical deriving from (C₁-C₁₂)alkane. In another particular embodiment, in the moiety of formula (I), L is a diradical deriving from (C₁-C₆)alkane. In one embodiment, in the moiety of formula (I), FG₁ and FG₂ are each a diradical independently selected from the group consisting of the diradicals of formula (IV), (V), (VI), (VII) and (IX). In another embodiment, in the moiety of formula (I), FG₁ and FG₂ are each a diradical independently selected from the group consisting of the diradicals of formula (IV), (VI) and (VII). In a particular embodiment, in the moiety of formula (I), FG₁ and FG₂ are a diradical of formula (VI). In one embodiment, X is O. In another embodiment, R₁ is H.

In one embodiment, in the moiety of formula (II), n is 0. In the moiety of formula (II), when n is 0, FG₁ is selected from the group consisting of the diradicals of formula (III), (IV), (V) and (VI), where X is O. In another embodiment, in the moiety of formula (II), n is comprised from 1 to 5. In the moiety of formula (II), when n is comprised from 1 to 5, FG₁ is a diradical selected from the diradicals of formula (IV), (V), (VI), (VII) and (IX). Particularly, in the moiety of formula (II), when n is comprised from 1 to 5, FG₁ is selected from the diradicals of formula (IV), (VI) and (VII).

In a particular embodiment, the biomolecule is bound to the surface of the shell through a moiety of formula (I), wherein n is an integer comprised of from 1 to 5; and FG₁ and FG₂ are independently selected from the diradicals of formula (IV), (VI) and (VII) where X is O. In another embodiment the nanoparticle is bound to the shell through a moiety of formula (II), wherein n is an integer comprised of from 1 to 5; and FG₁ is selected from the diradicals of formula (IV), (VI) and (VII) where X is O. In another particular embodiment, the nanoparticle is bound to the shell through a moiety of formula (II), wherein n is 0 and FG₁ is selected from the diradicals of formula (III), (IV), (VI) or (V), wherein X is O.

As mentioned above, the biomolecule comprised in the nanoparticle of the invention is selected from a peptide, a nucleic acid, a carbohydrate and a lipid which comprises a cleavage site that is susceptible of being cleaved by a target hydrolytic enzyme. Target hydrolytic enzymes are selected from those classified as EC 3 in the EC number classification of enzymes. In one embodiment, the biomolecule is a nucleic acid containing a cleavage site susceptible of being cleaved by a nuclease. In another embodiment, the biomolecule is a carbohydrate containing a cleavage site susceptible of being cleaved by a glycosyl hydrolase. In another embodiment, the biomolecule is a lipid containing a cleavage site susceptible of being cleaved by a lipase. In another embodiment, the biomolecule is a peptide containing a cleavage site susceptible of being cleaved by a protease. In another embodiment the sequence of said peptide comprises 3 to 12 aminoacids. In a particular embodiment, the biomolecule is a peptide containing a cleavage site susceptible of being cleaved by trypsin. In another particular embodiment, the biomolecule is a peptide comprising the following sequence: Cys-Lys-Arg-Val-Lys (SEQ ID NO: 1), which contains a cleavage site for trypsin. Trypsin specifically cleaves proteins at the carboxyl side (or "C-terminal side") of the amino acids lysine and arginine within a peptide chain, except when either is bound to a C-terminal proline. Peptides, nucleic acids, carbohydrates and lipids designed to contain a cleavage site for a hydrolytic enzyme can be prepared according to known methods. In particular, peptides designed to contain a cleavage site for a hydrolytic enzyme can be prepared by solid-phase synthesis.

The biomolecule used in the invention is bound to a PL label, wherein said label is bound to the part of the biomolecule which detaches from the nanoparticle after cleavage of said biomolecule by a hydrolytic enzyme through a diradical of formula FG₃ which is selected from the group consisting of the diradicals of formula (III), (IV), (V), (VI), (VII), (VIII) and (IX) defined above. In particular embodiments, FG₃ is selected from the diradicals of formula (IV), (VII) and (VIII). More particularly, FG₃ is a diradical of formula (VII) wherein R₁ is H. Non-limitative PL labels in the sense of the present invention are rhodamine, and rhodamine-derivatives such as tetramethylrhodamine (TMR), carboxytetramethyl-rhodamine (TAMRA), Carboxy-X-rhodamine (ROX), tetramethylrhodamine 5-(or 6)-isothiocyanate (TRITC), 5-(or 6)-carboxytetramethylrhodamine succinimidyl ester (NHS-rhodamine), tetraethylrhodamine hydrochloride (Rhodamine B), Xanthylium, 9-(2-(ethoxycarbonyl)phenyl)-3,6-bis(ethylamino)-2,7-dimethyl, chloride (Rhodamine 6G), sulforhodamine 101 sulfonyl chloride (Texas Red), and 5-[(5-carboxypentyl)sulfamoyl]-2-[6-(diethylamino)-3-(diethyliminiumyl)-3H-xanthen-9-yl]benzene-1-sulfonate (Rhodamine Red-X). In a particular embodiment, the label is TAMRA. In another particular embodiment, the nanoparticle comprises a peptide containing a cleavage site susceptible of being cleaved by a protease, particularly trypsin, bound to TAMRA. In another particular embodiment, the TAMRA-labelled peptide is defined by the sequence Cys-Lys-Arg-Val-Lys-TAMRA (SEQ ID NO: 2).

In another particular embodiment the nanoparticle of the invention contains at least: (a) a moiety of formula (I), wherein n is an integer comprised between 1 to 5, FG₁ and FG₂ are independently selected from the diradicals of formula (IV), (VI) and (VII), and X is O, (b) a biomolecule which is a peptide comprising a cleavage site that is susceptible of being cleaved by trypsin, the sequence of said peptide comprising from 3 to 12 aminoacids, and (c) a PL label bound to said peptide through a diradical selected from the diradicals of formula (IV), (VII) and (VIII). In another particular embodiment the nanoparticle of the invention contains at least: (a) a moiety of formula (I), wherein n is an integer comprised between 1 to 5, FG₁ and FG₂ are independently selected from the biradicals of formula (IV), (VI) and (VII), where X is O, (b) a biomolecule which is a peptide of SEQ ID NO:1, and (c) a PL label bound to said peptide through a diradical of formula (VII) wherein R₁ is H.

In another particular embodiment the nanoparticle of the invention comprises at least one structure of formula (X) bound to the surface of the shell, wherein n is an integer from 1 to 5, m is an integer from 1 to 6, and PL is rhodamine or a rhodamine derivative.

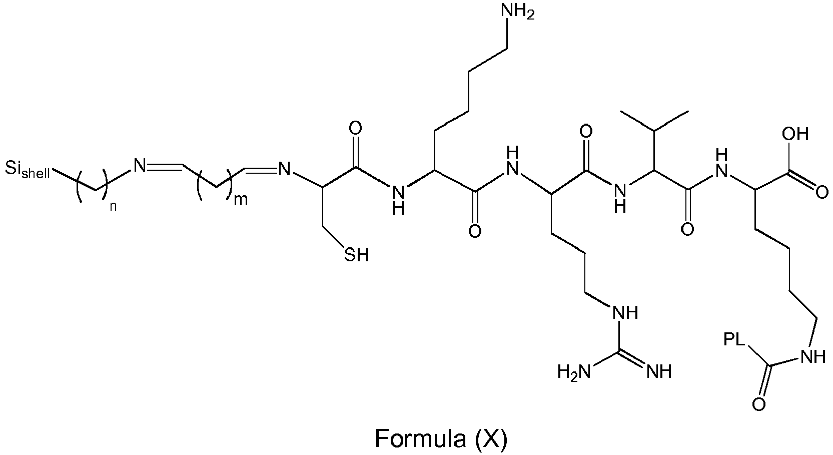

In another particular embodiment the nanoparticle of the invention comprises at least one structure of formula (X') bound to the surface of the shell. In this embodiment the biomolecule is a TAMRA-labelled peptide of SEQ ID NO: 2.

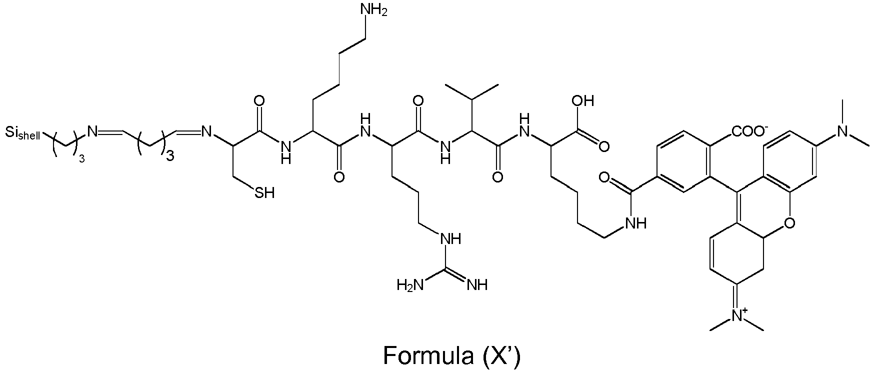

Generally, the ratio of PL-labelled biomolecule/nanoparticle is comprised from 0.1/1 to 100/1, i.e. 0.1 to 100 PL-labelled biomolecules are comprised in one nanoparticle. In one embodiment, the ratio of PL-labelled biomolecule/nanoparticle is comprised from 50/1 to 100/1, i.e. 50 to 100 PL-labelled biomolecules are comprised in one nanoparticle. In another embodiment, the ratio is comprised from 70/1 to 90/1. In a particular embodiment, the biomolecule is a TAMRA-labelled peptide comprising a trypsin cleavage site as defined above (SEQ ID NO: 2), and the appropriate ratio of peptide/nanoparticle is about 83/1.

In another embodiment, the ratio of PL-labelled biomolecule/nanoparticle is comprised from 0.1/1 to 50/1. In another embodiment, the ratio is comprised from 0.1/1 to 5/1. In another embodiment, the ratio is comprised from 0.3/1 to 3/1. In another particular embodiment, the biomolecule is a TAMRA-labelled peptide comprising a trypsin cleavage site as defined above (SEQ ID NO: 2), and the appropriate ratio of peptide/nanoparticle is about 1/1.

In one embodiment, the average distance between the label and the QD2 is comprised from 7 to 22 nm and the PL emission of the QD2 and the PL absorption of the label or the PL emission of the label and the PL absorption of the QD2 have a spectral overlap comprised from 40 to 80% area/area. This distance and spectral overlap are particularly adequate for efficient FRET in the nanoparticles of the invention. Additionally, the average distance between the first and the second QD populations can be comprised from 25 to 100 nm. Such distance can effectively avoid FRET processes between the two QD populations, and also between the QD1 and the label in the nanoparticles of the invention. Efficient FRET can also be precluded between the first and second QD populations, or between the QD1 and the label, when the PL emission spectrum of the donor and the PL absorption spectrum of the acceptor do not have a significant spectral overlap, even if the average distance is below 25 nm. Thus, in addition to the above distance between the QD1 and QD2, or as an alternative thereto, the PL emission spectrum of the QD1 and the PL absorption spectrum of the label have a spectral overlap comprised from 0 to 30% area/area, which effectively precludes FRET between the first QD population (as donor) and the label (as acceptor), or the PL emission spectrum of the label and the PL absorption spectrum of the first QD population have a spectral overlap comprised from 0 to 30% area/area, which effectively precludes FRET between the label (as donor) and the QD1 (as acceptor). Also, in addition to a distance comprised from 25 to 100 nm between the QD1 and PL, or as an alternative thereto, the PL emission spectrum of the QD1 and the PL absorption spectrum of the QD2 have a spectral overlap comprised from 0 to 30% area/area, or the PL emission spectrum of the QD2 and the PL absorption spectrum of the QD1 have a spectral overlap comprised from 0 to 30% area/area.

In a particular embodiment, the average distance between the label and the QD2 is comprised from 10 to 18 nm, for example, 10, 11, 12, 13, 14, 15, 16, 17 or 18 nm. In another particular embodiment the spectral overlap between the PL emission spectrum of the QD2 and the PL absorption spectrum of the label or between the PL emission spectrum of the label and the PL absorption spectrum of the QD2 is comprised from 50 to 80% area/area, or between 60 and 80% area/area, or between 70 and 80% area/area. Further, in a particular configuration of the nanoparticle of the invention, the QD2 is the donor and the PL label is the acceptor in the FRET process. However, the reverse configuration is also possible.

In a particular embodiment the nanoparticle of the invention has the following characteristics: the average distance between the PL label and the QD2 is comprised from 12 to 16 nm, the PL emission spectrum of the QD2 and the PL absorption spectrum of the label have a spectral overlap comprised from 60 to 80% area/area, and the average distance between the label and the QD1 is comprised from 40 to 90 nm.

In a preferred embodiment of the invention, the QD1 and QD2 populations comprise CdSe nanocrystals.

In one embodiment of the invention, the QD1 has PL emission wavelength comprised from 600 to 700 nm, the QD2 has PL emission wavelength comprised from 500 to 600 nm, and the label has PL absorption wavelength comprised from 450 to 650 nm. In another embodiment, the QD1 has PL emission wavelength comprised from 630 to 680 nm, the QD2 has PL emission wavelength comprised from 520 to 560 nm, and the label has PL absorption wavelength comprised from 500 to 620 nm. In another embodiment, the QD1 has PL emission wavelength comprised from 640 to 670 nm, particularly 660 nm, the QD2 has PL emission wavelength comprised from 530 to 550 nm, particularly, 540 nm, and the label has PL absorption wavelength comprised from 550 to 600 nm, particularly 580 nm.

The nanoparticles of the invention constitute ratiometric biosensors particularly appropriate for quantifying hydrolytic enzyme activity. Enzymatic digestion of biomolecules is highly specific, each hydrolytic enzyme cleaving particular bonds within the biomolecule. Thus, in one embodiment of the present invention the nanoparticles are used for quantifying trypsin in a sample. To this end, the nanoparticle of the invention comprises a peptide which comprises a site susceptible of being cleaved by trypsin.

The method for quantifying a hydrolytic enzyme according to the invention proceeds as follows. In the resting state (which is defined as the absence of a hydrolytic enzyme), FRET energy transfer is established between the second QD population and the label upon excitation of the nanoparticle of the invention. By excitation it is understood subjecting the nanoparticles to a light source which can make the first and second QD populations emit a PL emission. Said excitation light source can be comprised, for instance, from 375 to 515 nm. The PL intensities of the two QD populations can be determined by conventional detection means, for example, a spectrophotometer (or a luminescent spectrophotometer, a fluorometer, etc). Three different signals will be detected because the QD1, QD2 and label in the nanoparticles of the invention have different emission spectra. When a nanoparticle that contains a biomolecule comprising a cleavage site for a hydrolytic enzyme is contacted with said enzyme, cleavage of the biomolecule takes place. Said cleavage interrupts FRET between the QD2 and the PL label, resulting in a detectable change in PL intensity of the system. When the nanoparticles according to the invention are contacted with the enzyme, the variation in PL intensity is proportional to the enzyme's hydrolytic activity (i.e. can be correlated with enzyme concentration). Further, since no FRET interactions are established between the QD1 and the other components of the nanoparticle, the PL intensity of the QD1 remains constant and provides for an internal reference for the quantification. Said internal reference significantly contributes to the sensitivity and robustness of the quantification method, particularly because it is unaffected by the environmental conditions (PL intensity of QD1 inside the core remains constant regardless of pH, temperature or any other environmental conditions).

The method of the invention is illustrated in FIG 2 for trypsin quantification. In this particular case, when trypsin cleaves the peptide bound to the nanoparticle, FRET between the QD2 and the PL label is interrupted resulting in a detectable change in PL intensity of the QD2 (because the label no longer absorbs the emitted signal, provoking an increase in the emission signal of the QD2). It is to be noted, however, that trypsin quantification is only a particular embodiment which does not limit the scope of the invention, since the quantification method disclosed herein can be applied in the same way to quantify other hydrolytic enzymes.

In the method of the invention the relation between PL intensities of the second and the first QD populations is determined and correlated with enzyme quantity. This correlation can be determined by calibration of the system with samples of known enzyme concentration, that is, by determining the relation between the PL intensities of the second and first QD populations in the absence of enzyme and in the presence of known enzyme concentrations. An unknown enzyme concentration can then be extrapolated only by determining the relation between the detected PL intensities of the second and first QD populations in the problem sample. Calibration of a system, which makes use of particular nanoparticles according to the invention, is explained in the examples below for trypsin quantification. The same calibration procedure can be used to determine the correlation of other hydrolytic enzymes with the PL intensities of the QD populations of analogous nanoparticles.

The high specificity of the cleavage site within the biomolecule, the high performance of FRET and the internal reference all contribute to render the method of the invention highly sensitive, robust and accurate. Usually, the result can be obtained very quickly, only minutes after mixing of the nanoparticles with the analyte. When the present method is used for diagnosis purposes, for example, for the diagnosis of CF, results are obtained in a shorter time and with less manipulation than with conventional immunochemical methods. Additionally, the present bioassay can be downscaled to be applied at the point of use. Portable spectrophotometers are known in the art and could be adapted for use with the method of the invention.

As is shown in the examples below, the inventors have determined trypsin concentration in the faeces of CF patients with the method of the invention and found that said patients have trypsin concentrations comprised from 0 to 90 µg/g. Additionally, the method of the invention can be used to determine a subject's phenotype for the CFTR gene by quantifying the amount of trypsin in the faeces. Subjects having two functional copies of the CFTR gene show faecal trypsin concentration comprised from 160 to 340 µg/g, while subjects having one mutated (non-functional) copy of the CFTR gene show faecal trypsin concentration comprised from 91 to 349 µg /g. This is of interest because CF has an autosomal recessive pattern of inheritance. Most people without CF have two functional copies of the CFTR gene, and both copies must be non-functional for CF to develop. Thus, faecal trypsin concentration comprised from 91 to 159 µg/g indicates that the subject is heterozygotic for the CFTR gene (has one functional and one non-functional copy of the gene). The patient is then classified as carrier for CF, i.e., does not develop the disease but the descendants could, according to an autosomal recessive pattern of inheritance. In contrast, faecal trypsin concentration above 340 µg/g is indicative of two functional copies of CFTR gene, i.e., indicative of non-carrier status. As mentioned above, faecal trypsin concentration from 0 to 90 µg/g indicates that the subject suffers from CF and therefore has two non-functional copies of the CFTR gene. Thus one embodiment the invention provides a method as defined above for classifying a subject as homozygotic carrier of CF disease, heterozygotic carrier of CF disease or non-carrier of CF disease, wherein a feacal trypsin concentration comprised from 0 to 90 µg/g is indicative of homozygotic carrier of CF disease, faecal trypsin concentration comprised from 91 to 159 µg/g is indicative of heterozygotic carrier of CF disease and faecal trypsin concentration comprised above 340 µg/g is indicative of non-carrier of CF disease.

The disclosure also refers to a kit for quantifying a hydrolytic enzyme in a sample comprising a nanoparticle as defined above together with instructions for the quantification of the hydrolytic enzyme. In a particular embodiment the invention provides a kit for the diagnosis of CF. Said kit comprises nanoparticles wherein the biomolecule comprises a cleavage site for trypsin as defined in some embodiments above, as well as instructions for the quantification of trypsin in a biological sample. Said instructions are, for example, instructions for performing the test, taking the measure, calibrating the system, or even a pre-calculated calibration curve for direct quantification as the one provided in the examples below. The kit can additionally contain other reagents or means for performing the test, for example, buffers, assay solution, positive and negative controls, stop solution (a solution for stopping the hydrolytic reaction), disposable cuvettes, microtiter plates, or the like. The kit can also include means for self-calibration of the quantification method, such as serial trypsin dilutions. The kit contains a suspension of nanoparticles of the invention in a buffer solution. The buffer solution usually has neutral pH. In a particular embodiment the buffer is phosphate buffer solution (PBS). In another embodiment the nanoparticles of the invention are deposited, immobilised, or coated, on a solid support. For example, the nanoparticles of the invention may be immobilised, or coated, or deposited on a membrane, a glass plate, a polystyrene plate, a microtiter plate, etc. The nanoparticles may be directly immobilised in the solid support and optionally covered by a hydrogel. Alternatively, the nanoparticles can be included in a hydrogel or sol-gel matrix previous to immobilisation on the solid support. Such nanoparticle-preloaded supports are then directly used as substrates for the method of the invention. For instance, the sample containing trypsin is directly added to a nanoparticle-preloaded microtiter plate and, after a short time, the read-out signals are determined. This format has the advantage of assaying multiple samples at the same time.

The invention also provides a method for preparing the nanoparticles. In one embodiment, the invention refers to a method for preparing a nanoparticle as defined above, which comprises (i) forming a core comprising a first population of silica embedded QDs by the reverse microemulsion method, (ii) forming a shell containing a second population of silica embedded QDs by subjecting the core comprising a first population of silica embedded QDs of step (i) to reverse microemulsion method, (iii) recovering the core-shell nanoparticle obtained in step (ii), optionally (iv) functionalising the surface of the shell, and (v) adding a biomolecule selected from a peptide, a nucleic acid, a carbohydrate or a lipid which comprises a cleavage site that is susceptible of being cleaved by a hydrolytic enzyme and is bound to a photoluminescent label that is bound to the part of the biomolecule which detaches from the nanoparticle after cleavage of said biomolecule by a hydrolytic enzyme through a diradical of formula FG₃ as defined in claim 1; whereby the biomolecule is bound to the surface of the shell through a moiety selected from the moieties of formula (I) and (II) as defined in claim 1. Said method first comprises forming a core which contains a QD1 embedded in silica (QD1@SiO2). The reverse microemulsion method can be used for this purpose. The obtained QD1@SiO2 are consequently used as starting material for a second reverse microemulsion step, in which a QD2 is added to the QD1@SiO2 core. The result of this second reverse microemulsion step is the formation of a shell around the QD1@SiO2, said shell containing a QD2, also embedded in silica (QD1@SiO2@QD2@SiO2). The surface of the so-formed core/shell structure has free functional groups, for example, hydroxy or aldehyde functional groups, depending on the reagent used during the reverse microemulsion step, that are susceptible of reacting with further compounds, said compounds being consequently bound to the surface of the shell. However, if a different reactivity of the surface is desired, functionalization of said surface is possible as will be explained below. After recovering and washing the core/shell structures, with or without further functionalization, the next step in the method of the invention is to add a biomolecule that is bound to a photoluminescent label and that comprises a cleavage site susceptible of being cleaved by a hydrolytic enzyme.

The reverse microemulsion method is known in the art for preparing silica-embedded QDs (Ma et al, "Multiplexed color encoded silica nanospheres prepared by stepwise encapsulating quantum dot/SiO2 multilayers", Chem. Commun., 2011, vol. 47, pp. 7071-7073). It basically comprises adding, under vigorous stirring, QDs and a silica-providing compound into a liquid system containing an organic solvent and a non-ionic surfactant. After a time comprised from 20 to 30 min, ammonia is injected and the resulting emulsion is stirred for a time comprised from 2 to 24 h. For the preparation of the nanoparticles of the invention this reverse microemulsion step is first performed with the QD1 giving rise to the beads that will constitute the core. Then, addition of the QD2, more silica-providing compound and, later on, ammonia to the liquid system subsequently condenses to form the shell of silica-embedded QD2 around the cores.

Non-limiting silica-providing compounds for use in the present invention are TEOS (tetraethyl orthosilicate) and TEAS (triethylalkoxy aldehyde silane). Non-limiting organic solvents for use in the reverse microemulsion method are cyclohexane, hexane, pentane, heptane, methanol and ethanol. Non-limiting non-ionic surfactants for use in the invention are NP-7 (nonylphenol ethoxylate), IGEPAL CA-630 (Octylphenyl-polyethylene glycol), NP-40 (nonyl phenoxypolyethoxylethanol), Triton X100 (polyethylene glycol p-(1,1,3,3-tetramethylbutyl)-phenyl ether), and polysorbate 80.

When the second reverse microemulsion step is completed, the core-shell structures are recovered by conventional means, for example, by filtration or centrifugation, and washed one or more times. The washing step is usually performed with an organic solvent, for example, ethanol, methanol or acetone.

The surface of the so-formed core-shell structure has free functional groups. Said functional groups depend on the silica-providing compound that is used in the second reverse microemulsion step. For example, when TEOS is used, the functional group at the surface of the shell is hydroxy. When TEAS is used, the functional group is aldehyde. Thus, the reactivity of the surface of the shell can be predetermined by the preparation method.

Taking advantage of said reactivity, the biomolecule may be directly connected to the core/shell structure (without functionalization of the surface). This yields a nanoparticle wherein the biomolecule is bound to the surface of the shell through a moiety of formula (II), wherein n is 0 and FG₁ is selected from the diradicals of formula (III), (IV), (V) or (VI) as defined above, wherein X is O. A suitable method for obtaining a nanoparticle according to this embodiment comprises reacting the biomolecule containing a functional group suitable for reacting with the functional group of the core/shell structure as obtained above. Functional groups that can react with the surface of the core/shell structure as obtained above are hydroxyl, carboxyl, amine, and phosphoryl groups. Phosphoryl groups may be, in a non-limitative way, phosphate esters or phosphate diesters.

However, as has been mentioned above, the surface of the shell may be optionally functionalised before adding the biomolecule. This yields nanoparticles comprising a moiety of formula (I) or (II), where n is comprised from 1 to 10.

In one embodiment the biomolecule is bound to the surface of the shell through a moiety of formula (II), wherein n is comprised from 1 to 10. A suitable method for the preparation of a nanoparticle according to this embodiment comprises the following steps:
(a) Reacting the core/shell structure with a compound of formula XI 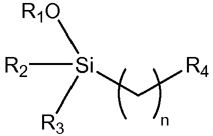 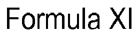
   wherein R₁ is H or (C₁-C₃)alkyl,
   R₂ and R₃ are independently selected from OR₅ or (C₁-C₃)alkyl,
   R₅ is selected from H or (C₁-C₃)alkyl,
   R₄ is selected from NH₂, SH, OH and CO₂H, and
   n is an integer selected from 1 to 10.

   This reaction is performed under stirring in the presence of an organic solvent, such as toluene, at room temperature and stirring the emulsion for a time comprised from 15 to 30 h, for instance 24 h. In particular embodiments, 3-(trihydroxysilyl)propyl methylphosphonate (THPMP) can be also added to this reaction in order to avoid aggregation of the resulting nanoparticles. In some embodiments, R₂ and R₃ in the compound of formula XI are independently OR₅. In particular embodiments, the compound of formula XI is selected from aminopropyl trimethoxysilane (APTMS), aminopropyl triethoxysilane (APTES), 3-mercaptopropyl trimethoxysilane (MPTMS) and 3-mercaptopropyl triethoxysilane (MPTES);
(b) reacting the product of (a) with a biomolecule selected from a peptide, a nucleic acid, a carbohydrate or a lipid which comprises a site susceptible of being cleaved by a hydrolytic enzyme. This step is performed under conditions appropriate for forming of a covalent bond between a free functional group comprised in the biomolecule and R₄. In a particular embodiment the biomolecule is a PL-labelled biomolecule.

Thus, step (b) comprises reacting the product of (a) with a biomolecule selected from a peptide, a nucleic acid, a carbohydrate or a lipid, wherein said biomolecule comprises a site susceptible of being cleaved by a hydrolytic enzyme, and a photoluminescent label that is bound to the part of the biomolecule which detaches from the nanoparticle after cleavage of said biomolecule by a hydrolytic enzyme through a diradical of formula FG₃ as previously defined, whereby the biomolecule is bound to the surface of the shell through a moiety selected from the moieties of formula (I) and (II) as previously defined.

In yet another embodiment, the biomolecule is bound to the surface of the shell through a moiety of formula (I), wherein n is comprised from 1 to 10. This embodiment also implies the functionalization of the surface of the shell previous to binding of the biomolecule. A suitable method for the preparation of a nanoparticle according to this embodiment comprises the step (a), (b') and (c), wherein step (a) is as defined above and steps (b') and (c) are as follows:
(b') Reacting the product of (a) with a compound of formula XII 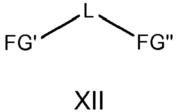 wherein L is a diradical selected from the diradicals deriving from a compound selected from (C₁-C₁₂)alkane, (C₁-C₁₂)alkene, (C₁-C₁₂)alkyne, (C₃-C₆)cycloalkane and benzene, and FG' and FG" are independently selected from -OH, -CO₂H, -OP(O)(OR')₂, -CHO, -SO₂H, -SO₂Cl and -SH where R' is H or (C₁-C₆)alkyl.
   The compound of formula XII can be, for instance, a (C₁-C₁₂)diacid, a (C₁-C₁₂)diamine, a (C₁-C₁₂)hydroxyamine, a (C₁-C₁₂)hydroxyacid, a (C₁-C₁₂)diol, a (C₁-C₁₂)dialdehyde, a diketone or an aminoacid. Typical non-limiting compounds of formula XII are (C₁-C₁₂)polyethyleneimine, (C₁-C₁₂)mercaptoalkanoic acids (such as mercaptoacetic acid, mercaptopropionic acid and mercaptoundecanoic acid), thioglycolic acid, and (C₂-C₁₂)dialdehydes. In a particular embodiment the compound of formula XII is a (C₁-C₁₂)dialdehyde, such as glutaraldehyde.
(c) reacting the product of (b') with a biomolecule selected from a peptide, a nucleic acid, a carbohydrate or a lipid which comprises a site susceptible of being cleaved by a hydrolytic enzyme. This step is performed under conditions appropriate for forming of a covalent bond between a free functional group comprised in the biomolecule and the FG". In a particular embodiment the biomolecule is a PL-labelled biomolecule.

Thus, step (c) comprises reacting the product of (b') with a biomolecule selected from a peptide, a nucleic acid, a carbohydrate or a lipid, wherein said biomolecule comprises a site susceptible of being cleaved by a hydrolytic enzyme, and a photoluminescent label that is bound to the part of the biomolecule which detaches from the nanoparticle after cleavage of said biomolecule by a hydrolytic enzyme through a diradical of formula FG₃ as previously defined, whereby the biomolecule is bound to the surface of the shell through a moiety selected from the moieties of formula (I) and (II) as previously defined.

This method is advantageous as this allows fine-tuning the reactivity of the core/shell with the biomolecule and therefore allows for higher versatility of the system. When functionalising the surface of the shell it must be taken into account that the added moiety must not exceed a certain length, such that the distance of the second QD population and the label is not above 25 nm, so that FRET can be established.

Typically, the biomolecule that comprises a cleavage site susceptible of being cleaved by a hydrolytic enzyme is bound to the PL label previous to the step of adding said biomolecule to the surface of the shell. Appropriate PL labels have been mentioned above. Labelling of peptides, carbohydrates, lipids and/or nucleic acids with PL compounds, particularly rhodamine and rhodamine derivatives is known in the art and some PL-labelled peptides are commercially available. Labelled peptides can be prepared by either modifying isolated peptides or by incorporating the label during solid-phase synthesis. Three strategies are used to label peptides with dyes: 1) Labelling during synthesis of peptide. Dyes that are not damaged by unblocking procedures are incorporated onto the amino terminus of the peptide chain; 2) Synthetic peptides can be covalently modified on specific residues and labels incorporated following synthesis; and 3) Synthetic peptides may be covalently labelled by amine- or thiol reactive protein labels.

Fluorophores can be conjugated to the N-terminus of a resin-bound peptide before other protecting groups are removed and the labelled peptide is released from the resin. Amine-reactive fluorophores are used in about 5-fold molar excess relative to the amines of the immobilized peptide. Another possibility is the use of fluorescence or chromophore-labelled amino acids to incorporate labels at specific sites of peptides. Labelling can also be achieved indirectly by using a biotinylated amino acid. If, for example, Fmoc-Lys(biotinyl)-OH is used in peptide synthesis, the biotin group allows specific binding of streptavidin or avidin-conjugate to that site.
Following the routine synthesis procedure, peptides can also be labelled by practically all labels used for protein labelling. This means mainly amine reactive labels, or thiol reactive labels, if a cystein has been used for the peptide. Whereas the common standard procedures for protein labelling are based on aqueous solutions of target proteins, labelling peptides in organic solvents like DMSO or DMF requires specific modifications. Use of triethylamine or other tertiary amines can be added to ensure that the target amino groups of the peptide are deprotonated, which is required for the labelling procedure. In the nanoparticle described below, the labelled peptide with SEQ ID NO: 2 was obtained by the formation of a peptidic bond between the TAMRA dye and the amino group of the terminal lysine side chain prior to peptide synthesis. Methods for the formation of peptidic bonds are well known in the art.
A number of different nanoparticles according to the invention may be obtained by the above method depending on the particular silica-providing compounds, functionalization compounds to form the moiety, PL labels and biomolecules comprising a cleavage site that is susceptible of being cleaved by a hydrolytic enzyme, as well as the stoichiometry of the reactants. Thus the disclosure also provides a nanoparticle obtainable by the processes described in this invention, in particular the process comprising the following steps:
(i) forming a core comprising a first population of silica embedded QDs by the reverse microemulsion method, (ii) forming a shell containing a second population of silica embedded QDs by subjecting the core comprising a first population of silica embedded QDs of step (i) to reverse microemulsion method, (iii) recovering the core-shell nanoparticle obtained in step (ii), and optionally functionalising the surface of the shell, and (iv) adding at least one biomolecule selected from a peptide, a nucleic acid, a carbohydrate or a lipid, wherein each biomolecule comprises a cleavage site that is susceptible of being cleaved by a hydrolytic enzyme, and a photoluminescent label that is bound to the part of the biomolecule which detaches from the nanoparticle after cleavage of said biomolecule by a hydrolytic enzyme through a diradical of formula FG₃ as previously defined; whereby the biomolecule is bound to the surface of the shell through a moiety selected from the moieties of formula (I) and (II) as previously defined. It is provided a nanoparticle obtainable by forming a core/shell structure by the steps (i)-(iii) as previously defined and further functionalising the surface of the shell and adding a PL-labelled biomolecule as defined in steps (a)-(b) as mentioned above. It is provided a nanoparticle obtainable by forming a core/shell structure by the steps (i)-(iii) and further functionalising the surface of the shell and adding a PL-labelled biomolecule as defined in steps (a)-(b')-(c) as mentioned above. It is provided a nanoparticle obtainable by forming a core/shell structure by the steps (i)-(iii) and further functionalising the surface of the shell and adding a PL-labelled biomolecule as defined in steps (a)-(b')-(c) as mentioned above, wherein the compound of formula XI is APTMS, the compound of formula XII is glutaraldehyde and the PL-labelled biomolecule is a PL-labelled peptide of SEQ ID NO: 2.
For the purposes of the invention, the expressions "obtainable", "obtained" and similar equivalent expressions are used interchangeably and, in any case, the expression "obtainable" encompasses the expression "obtained".
Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration. Reference signs related to drawings and placed in parentheses in a claim, are solely for attempting to increase the intelligibility of the claim, and shall not be construed as limiting the scope of the claim.

### EXAMPLES

Materials. Trypsin (13.000-20.000 units/mg), Selenium powder (∼ 100 mesh, 99.99%), technical-grade trioctylphosphine (TOP, 90%), anhydrous toluene (99.8%), technical-grade trioctylphosphine oxide (TOPO, 90%), cadmium acetate dihydrate (98%), metal basis cadmium oxide (CdO, >99.99%,), technical grade 1-octadecene (90%), tetraethyl orthosilicate (TEOS, 99.999 methal basis), 3-aminopropyl-trimethoxysilane (APTMS, 97%), (3-trihydroxysilyl) propyl methylphosphonate (THPMP), phosphate buffer solution (PBS) were purchased from Sigma-Aldrich. Ammonia was purchased from Fluka. All the rest of reagents were purchased with analytical grade. Peptides with SEQ ID NO: 2 and SEQ ID NO: 3 were purchased from Biotrend.

Synthesis of QD. CdSe QDs were obtained using TOPO-TOP as capping ligand. A selenium solution was prepared by mixing 0.4 g of selenium powder, 10 mL of TOP and 0.2 mL of anhydrous toluene and stirred under argon. A mixture of 20 g of TOPO and 0.25 g of cadmium acetate dihydrate was placed in a round-bottomed flask, stirred and heated to 150°C under argon and the temperature was increased to 320°C. When the mixture reached 320°C the selenium solution was quickly injected and then cooled to 270°C. The reaction was run for 5 or 45 minutes for the preparation of green emitting CdSe QDs (QD540) and red emitting CdSe QDs (QD660), respectively. After stopping the reaction, the samples were cooled and washed with ethanol and acetone three times and stored in 10 mL of chloroform.

Synthesis of the nanoparticles. First, silica-embedded QDs (QD@SiO2) nanospheres were prepared. Two populations of CdSe quantum dots were used: the first QD population with emission wavelength at 540 nm (green (2.7 nm), QD540) and the second QD population with emission wavelength at 660 nm (red (5.6 nm), QD660). Single and two colors quantum dots embedded silica nanospheres, i.e. QD540@SiO2 and QD660@SiO2@QD540@SiO2, respectively, were prepared by the reverse microemulsion method as detailed elsewhere (Ma et al, "Multiplexed color encoded silica nanospheres prepared by stepwise encapsulating quantum dot/SiO2 multilayers", Chem. Commun., 2011, vol. 47, pp. 7071-7073). 0.8 mL QDs660 (5 mM) as obtained above and 0.640 mL TEOS were introduced into a liquid system containing 15 mL of cyclohexane and 2.6 mL non-ionic surfactant NP-7 under vigorous stirring. After 30 min, 0.1 mL ammonia was injected. Then the microemulsion was stirred for 24 hours. Because the hydrolyzed TEOS and NP-7 molecules replaced the original hydrophobic ligands of CdSe QDs in this reverse microemulsion system, silica growth can occur around the hydrophobic QDs smoothly. It resulted in high luminescent QD660@SiO2 beads. Then, an addition of 0.8 mL (6 mM) QDs540 as obtained above and 0.1 mL TEOS to the liquid system subsequently condenses to form a thin shell of QDs540@SiO2 around the beads. An amine functionalization of the surface of the so-formed nanospheres was done adding 30 µL of APTMS and leaving to stir for 24 hours at room temperature. 80 µL of THPMP was also added to the reaction to avoid electrostatic aggregation of the nanoparticles. Then, the nanoparticles were centrifuged and washed with ethanol and stored in 5 mL PBS.

The surface of the QD540@SiO2 or QD660@SiO2@QD540@SiO2 nanospheres was further functionalized with glutaraldehyde. 2 mL of QD540@SiO2-APTMS or QD660@SiO2@QD540@SiO2-APTMS in PBS (taken from the stock of 5 mL prepared above) and 0.25 mL of glutaraldehyde (0.25 mg/mL) were mixed and shaked for 4 h at 37 °C. Then the solution was centrifuged to remove the unreacted glutaraldehyde and finally the pellet was resuspended in 5 mL PBS. The next step involved adding of the biomolecule, which was a TAMRA-labelled peptide containing a cleavage site for trypsin. Two different nanoparticles were prepared, each containing a different TAMRA-labelled peptide (the peptides were designed by BIOTREND: Pro-active peptide (NH2-Cys-Lys-Arg-Val-Lys-TAMRA, SEQ ID NO: 2) and In-active peptide (NH2-Cys-Lys-Pro-Val-Lys-TAMRA, SEC ID NO: 3). For binding of the peptides 500 µL of 400, 800 or 1200 µg/L TAMRA-labelled peptides was added to 2 mL of QD540@SiO2-APTMS-glutaraldehyde or QD660@SiO2@QD540@SiO2-APTMS- glutaraldehyde as obtained above and shaked for 20 hours at 4°C. After this time, the solution was centrifuged to remove the unbound peptides. In the end, the QD540@SiO2-APTMS-glutaraldehyde- SEQ ID NO: 2 or QD660@SiO2@QD540@SiO2-APTMS-glutaraldehyde- SEQ ID NO: 2 were resuspended and stored in 5 mL PBS.

Enzymatic digestion. A determined concentration of trypsin between 25-350 µg/L was added to 1 mL nanoparticles as obtained above (5-6 mM) and mixed for a period of time: from 30 sec to 120 min, at 37 °C. After this time, the mixture was heated up to 70°C and held for 1 h in order to denaturalize the enzyme and stop the reaction.

Characterization. The formation of the QDs@SiO2 nanospheres was directly visualized by transmission electron microscopy (TEM) using a JEOL 1011 microscope. The UV-Visible and fluorescence spectra were recorded using a 1 cm path length quartz cell in a Shimadzu UV Spectrophotometer 1700 and an Aminco-Bowman Series 2 luminescence spectrometer. The PL intensities at 540 and 660 nm were determined after excitation with a blue laser at 405 nm.

Trypsin quantification in human faeces. Human faeces of 11 subjects were analysed for trypsin concentration. 4 subjects had been previously diagnosed with CF and were confirmed as homozygotic for non-functional CFTR gene by genetic testing. The other 7 subjects did not have CF but were classified as heterozygotic for non-functional CFTR gene (i.e. CF carriers) by genetic testing. The four CF patients were included in assistance programs available at different hospitals in Barcelona for CF patients and were taking daily optimal doses of a pancreatic enzyme to increase protein absorption (8-10 capsules of Creon© 25,000 or 20 capsules of Creon© 10,000 daily). Written informed consent was obtained from all patients or their parents, and assent was also obtained from the minors.

1g of solid faeces from each subject (stored at -8 °C until further handling), was added to 3 mL of PBS (pH 7.2) and vigorously stirred until a homogenous suspension was obtained. For the measurements, the mixture was heated up to 37°C and 1 mL of 5-6 mM QD660@SiO2@QD540@SiO2-APTMS-glutaraldehyde- SEQ ID NO: 2 nanoparticles in PBS obtained as specified above was added. The reaction was run for 10 min, and then heated up to 70°C to stop the enzymatic activity by denaturalization for 1 h. Finally, the PL intensity at 540 and 660 nm of the samples was measured.

Statistics: Syntheses, enzymatic digestions, and quantification experiments were done at least three times each. The results obtained show 100% reproducibility.

### Results

Transmission Electron Microscopy (TEM) confirmed the formation of the core/shell structures. For a typical single color synthesis, the QD540@SiO2 beads displayed a diameter of 38 nm. When the beads were coated with a second population QDs@SiO2 layer, i.e. QD660@SiO2@QD540@SiO2, the average diameter of the nanoparticle increased to 77 nm.

FIG 3 shows the photoluminescence (PL) absorption-emission profiles of QD540@SiO2 nanospheres and the TAMRA dye. There is a blue shift in the PL of QD@SiO2 in comparison to the pristine QDs in solution, i.e. 520 nm versus 540 nm, as observed previously, yet the original fluorescence is recovered after the trypsin digestion as shown later. The TAMRA dye displays fluorescence emission at 575 nm. As it can be seen in FIG 3, the fluorescence emission from the QD540@SiO2 nanospheres (without any further surface functionalization at the silica nanosphere) overlaps with the absorption peak of TAMRA thus allowing the FRET process.

As mentioned in the methods section above, the next step was the assembly of the previously obtained beads with the TAMRA-labelled peptide. Two different labelled peptides were used, since one of them provides a control sample for the experiments. On one hand, the "pro-active" labelled peptide has the following sequence: 3'-NH2-Cys-Lys-Arg-Val-Lys-TAMRA-5'. Trypsin proteolytic activity is highly specific to the Lys-Arg-Val sequence and the enzyme digestions will cleave the peptide. On the other hand, the control sample, the "in-active" labelled peptide has the same chemical nature except for a change by Proline (Pro) instead of Arginine (Arg). Trypsin cannot digest the small "in-active" peptide and the fluorescence emission properties of the QDs will remain the same. The emission spectra of the QD540@SiO2 nanospheres was recorded when bound to different concentrations of the "pro-active" TAMRA-labelled peptide in order to see how the amount of dye affects the emission of QDs. It should be emphasized that increasing the amount of TAMRA-labelled peptides at the QD540@SiO2 nanospheres leads to a decrease in the emission intensity between λ = 500 nm and 550 nm range, which corresponds to the fluorescence emission wavelength of the quantum dots. The emission intensity drop is a direct evidence of efficient FRET between the QD540@SiO2 nanospheres and the TAMRA dye in the one-colour system, as reported previously in analogous FRET systems.

The one-colour system was incubated with trypsin for the peptide digestion, and the emission spectra were recorded. The spectroscopic profiles further demonstrated the existence of FRET processes between the QD540@SiO2 nanospheres and the "pro-active" TAMRA-labelled peptide. Moreover, the control sample with the "in-active" QD540@SiO2 system, as expected, did not show any significant change in the fluorescence emission.

The use of a single quantum dot fluorophore QD540@SiO2 allowed for the qualitative analysis of the trypsin proteolytic activity. However, this system could not provide for quantitative determination of the enzymatic activity. In order to obtain a quantitative determination, the inventors added a second population of QDs to the core of silica nanospheres with fluorescence emission at 660 nm (FIG. 4). Thus, the use of two QDs fluorophores with distinguishable emission spectra allowed to establishing a measurable ratio, namely, I₅₄₀/I₆₆₀. Moreover, the emission intensity at λ = 660 nm remains constant as the QD660 at the silica nanospheres does not influence the FRET process. The key feature in this two-colour system is that, due to the absorption properties of the nanocrystal QDs, both fluorophores can be excited at the same wavelength (λ = 405 nm), but only the nanocrystals in the outlayer emitting at 540 nm undergo FRET with the TAMRA dye. Despite a small overlapping between the emission of QD660 and the absorption of TAMRA, the distance between them however is around 38 nm thus holding off the energy transfer, meanwhile the distance and the overlapping between QD540 and the dye allow the FRET to occur. Using time-correlated single photon counting (TCSPC) and steady-state emission spectroscopy, the inventors correlated that the average distance between the QD540 and the TAMRA dye was 14.6 nm for an efficiency of 0.5238. The calculated R₀ (distance at which the efficiency of FRET is 50%) was 7.79 nm, and the average distance between QDs540 and TAMRA dye (r) was 7.657 calculated from the fluorescence. This was in good agreement with the value obtained from the lifetime measurements, i.e. 7.665 nm. In conclusion, all the prerequisites for a quantitative nanosensor were accomplished.

FIG. 5 illustrates the changes in the fluorescence emission of the QD660@SiO2@QD540@SiO2 nanoparticles in the presence of two different trypsin concentrations, 25 µg/L (FIG 5a) versus 350 µg/L (FIG 5b). Increasing the enzyme concentration led to faster recovery of the original emission at 540 nm.

The inventors performed the study of the enzymatic digestion using the "pro-active" labelled peptide in a wide range of trypsin concentrations (25-350 µg/L). FIG 6 shows the ratio between the PL emission peaks of both QD540 and QD660 (I₅₄₀/I₆₆₀) during enzymatic digestion for 120 minutes in different enzyme concentrations. There was a direct relationship between the enzymatic digestion rate and the enzyme concentration that allowed to unequivocally differentiating between the different enzyme concentrations used. In other words, the higher the trypsin concentration the shorter the time needed to reach a plateau in the kinetic measurements. Hence, analysing the trypsin activity at early digestion times using QD660@SiO2@QD540@SiO2 ratiometric system (particularly 10 minutes) the inventors could differentiate between the different trypsin concentrations as shown in FIG 7. As can be seen, the calibration curve proved to be highly linear (χ²=0.9947) and allowed to correlate the ratio between the PL intensities at 540 and 660 nm (I₅₄₀/I₆₆₀) of the samples with their trypsin concentration with good sensitivity.

This method was used for quantifying trypsin concentration in faecal samples from 4 CF patients (subjects C, F, I and K), and 7 healthy subjects which are heterozygotic for non-functional CFTR gene (subjects A, B, D, E, G, H and J). Trypsin concentration in these samples was determined based on the calibration curve plotted in the coordinates I₅₄₀/I₆₆₀ versus trypsin concentration as explained above. Results are shown in Table 1 and confirm that the faeces of the 4 CF patients contained faecal trypsin concentration below 90 µg/g faeces, while non-CF patients had faecal trypsin concentration above 90 µg/g.

**Table 1. Faecal trypsin concentration**

| **Subject** | **Trypsin (µg /g faeces)** | | | |
|---|---|---|---|---|
| | **as-measured** | | **corrected values** | |
| A | 171,98 | ± 3,081 | 171,98 | ± 3,081 |
| B | 121,92 | ± 1,504 | 121,92 | ± 1,504 |
| C | 129,19 | ± 4,053 | 40,05 | ± 1,256 |
| D | 217,01 | ± 8,341 | 217,01 | ± 8,341 |
| E | 188,04 | ± 7,277 | 188,04 | ± 7,277 |
| F | 121,15 | ± 4,694 | 10,9 | ± 0,422 |
| G | 168,58 | ± 9,997 | 168,58 | ± 9,997 |
| H | 158,85 | ± 7,767 | 158,85 | ± 7,767 |
| I | 111,89 | ± 8,272 | 34,69 | ± 2,564 |
| J | 133,41 | ± 3,043 | 133,41 | ± 3,043 |
| K | 128,93 | ± 4,905 | 50,28 | ± 1,913 |

It has to be taken into account that these particular CF patients were taking supplementary pancreatic enzymes (8-10 capsules of Creon© 25,000 or 20 capsules of Creon© 10,000 daily, respectively), and consequently, the additional enzymatic activity provided by such medication had to be subtracted from the as-measured trypsin concentration (see table 2).

**Table 2. Calculations for corrected faecal trypsin concentration in CF patients**

| | **C** | **F** | **I** | **K** |
|---|---|---|---|---|
| Medication | Creon 25,000 | Creon 10,000 | Creon 25,000 | Creon 25,000 |
| Trypsin/capsule¹ | 1,000 | 600 | 1,000 | 1,000 |
| Capsules/day | 9 | 20 | 9 | 8 |
| Total trypsin/day¹ | 9,000 | 12,000 | 9,000 | 8,000 |
| Normal trypsin^{1,2} | 13,000 | 13,000 | 13,000 | 13,000 |
| Extra trypsin from medication | 70% | 92% | 70% | 62% |
| Real trypsin in the body | 30% | 8% | 30% | 38% |
| [trypsin] as-measured (µg/g) | 129,19 | 121,15 | 111,89 | 128,93 |
| [trypsin] corrected (µg/g) | 40,05 | 10,9 | 34,69 | 50,28 |

| | | | | |
|---|---|---|---|---|
| ¹PhEur units ²Normal trypsin concentration in healthy subjects | | | | |

The overall results shown herein demonstrate that the nanoparticles of the invention provide for sensitive, specific and accurate trypsin activity quantification and that it is useful for CF diagnosis and prediction of subject's phenotype for the CFTR gen. The method is easy-to-use, fast, and robust and is thus very useful for the diagnostic of cystic fibrosis.

### REFERENCES CITED IN THE APPLICATION

Sapsford et al, "Monitoring of enzymatic proteolysis on a electroluminescent-CCD microchip platform using quantum dot-peptide substrates", Sensors and Actuators B, 2009, vol. 139, pp. 13-21.
Ma et al, "Multiplexed color encoded silica nanospheres prepared by stepwise encapsulating quantum dot/SiO2 multilayers", Chem. Commun., 2011, vol. 47, pp. 7071-7073.
Qiang Ma et al, "Multilayered, core/shell nanoprobes based on magnetic ferric oxide particles and quantum dots for multimodality imaging of breast cancer tumors", Biomaterials, 2012, vol. 33, pp. 8486-8494.

## Claims

1. A nanoparticle comprising
(i) a core comprising a first population of quantum dots (QDs) embedded in silica,
(ii) a shell comprising a second population of QDs embedded in silica,
(iii)at least one biomolecule selected from a peptide, a nucleic acid, a carbohydrate or a lipid which comprises a cleavage site that is susceptible of being cleaved by a hydrolytic enzyme, said biomolecule being bound to the surface of the shell through a moiety selected from the moieties of formula (I) and (II), 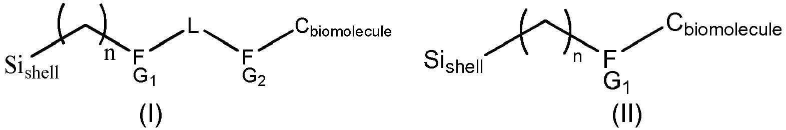
wherein Siₛₕₑₗₗ is a silicium atom comprised in the shell and C_{biomolecule} is a carbon atom comprised in the biomolecule;
n is an integer comprised of from 0 to 10;
FG₁ and FG₂ are each a diradical independently selected from the group consisting of the diradicals of formula (III), (IV), (V), (VI), (VII), (VIII) and (IX) 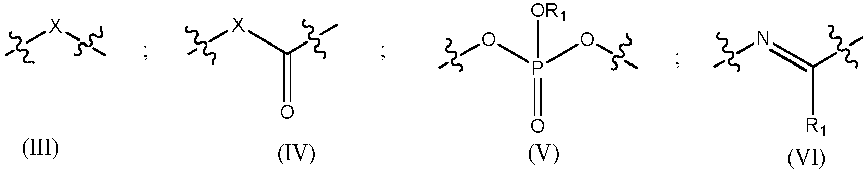 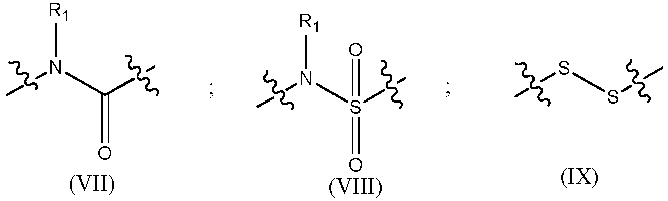
where X is S or O; R₁ is H or (C₁-C₁₂)alkyl;
L is a diradical selected from the diradicals deriving from a compound selected from (C₁-C₁₂)alkane, (C₁-C₁₂)alkene, (C₁-C₁₂)alkyne, (C₃-C₆)cycloalkane and benzene;
and
(iv)a photoluminescent label for each biomolecule, wherein the label is bound to the part of the biomolecule which detaches from the nanoparticle after cleavage of said biomolecule by a hydrolytic enzyme through a diradical of formula FG₃ which is selected from the group consisting of the diradicals of formula (III), (IV), (V), (VI), (VII), (VIII) and (IX) defined above;
wherein the first and second QD populations have different maximum photoluminescence emission wavelengths, and only the second QD population is susceptible of producing Förster resonance energy transfer (FRET) with the photoluminescent label.

2. The nanoparticle of claim 1 wherein the biomolecule is bound to the surface of the shell through a moiety of formula (I) wherein:
n is an integer comprised of from 1 to 5;
X is O; and
FG₁ and FG₂ are independently selected from the diradicals of formula (IV), (VI) and (VII).

3. The nanoparticle according to any of the claims 1 to 2 wherein FG₃ is selected from the diradicals of formula (IV), (VII) and (VIII).

4. The nanoparticle according to any of the claims 1 to 3 wherein the biomolecule is a peptide comprising a cleavage site that is susceptible of being cleaved by a proteolytic enzyme, the sequence of said peptide comprising from 3 to 12 aminoacids.

5. The nanoparticle according to any of the claims 1 to 4 wherein the biomolecule is a peptide comprising a cleavage site that is susceptible of being cleaved by trypsin.

6. The nanoparticle according to any of the claims 1-5, wherein
(i) the average distance between the photoluminescent label and the second QD population is comprised from 7 to 22 nm,
(ii) the photoluminescent emission spectrum of the second QD population and the photoluminescent absorption spectrum of the label have a spectral overlap comprised from 40 to 80% area/area; or the photoluminescent emission spectrum of the label and the photoluminescent absorption spectrum of the second QD population have a spectral overlap comprised from 40 to 80% area/area,
(iii) the average distance between the label and the first QD population is comprised from 25 to 100 nm and/or the photoluminescent emission spectrum of the first QD population and the photoluminescent absorption spectrum of the label have a spectral overlap comprised from 0 to 30% area/area; or the average distance between the label and the first QD population is comprised from 25 to 100 nm and/or the photoluminescent emission spectrum of the label and the photoluminescent absorption spectrum of the first QD population have a spectral overlap comprised from 0 to 30% area/area, and
(iv) the average distance between the first QD population and second QD population is comprised from 25 to 50 nm and/or the photoluminescent emission spectrum of the first QD population and the photoluminescent absorption spectrum of the second QD population have a spectral overlap from 0 to 30% area/area; or the average distance between the first QD population and second QD population is comprised from 25 to 50 nm and/or the photoluminescent emission spectrum of the second QD population and the photoluminescent absorption spectrum of the first QD population have a spectral overlap from 0 to 30% area/area.

7. The nanoparticle according to claim 6, wherein
(i) the first QD population has photoluminescent maximal emission wavelength comprised from 600 to 700 nm,
(ii) the second QD population has photoluminescent maximal emission wavelength comprised from 500 to 600 nm, and
(iii) the label has photoluminescent maximal absorption wavelength comprised from 450 to 650 nm.

8. The nanoparticle according to any of the claims 1 to 7 which comprises a structure of formula (X) bound to the surface of the shell 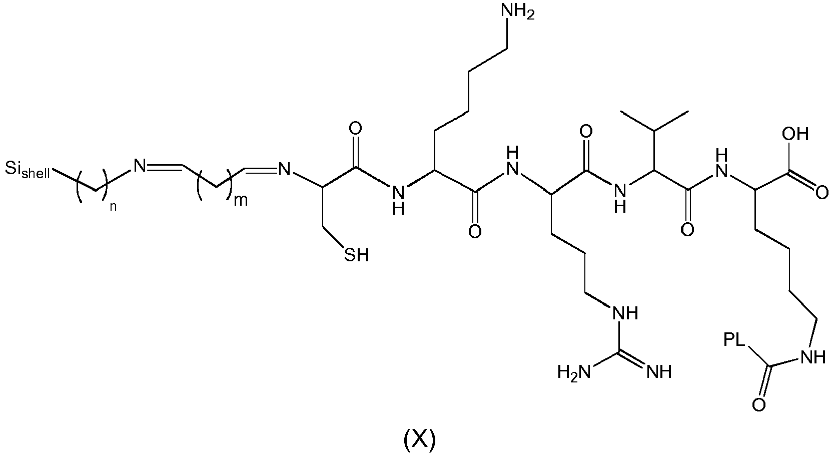
wherein m is an integer from 1 to 6; and
PL is a photoluminescent label selected from rhodamine or a rhodamine derivative.

9. The nanoparticle according to any of the claims 1-8, wherein the QDs are selected from the group consisting of CdSe, CdS, CdSe coated with ZnS (CdSe/ZnS) and CdS coated with ZnS (CdS/ZnS).

10. Use of a nanoparticle as defined in any of the claims 1-9, for quantifying a hydrolytic enzyme in a sample.

11. Use according to claim 10, wherein the nanoparticle comprises a peptide comprising a cleavage site susceptible of being cleaved by trypsin.

12. Use of a nanoparticle as defined in any of the claims 1-9, as a diagnostic agent for cystic fibrosis, wherein the nanoparticle is used for quantifying a hydrolytic enzyme in a sample, and it comprises a peptide comprising a cleavage site susceptible of being cleaved by trypsin.

13. A method for quantifying a hydrolytic enzyme in a sample comprising the steps of:
(i) contacting a nanoparticle according to any of the claims 1-9 with the sample,
(ii) determining the photoluminescent intensity of the first and second QD populations upon exposing the mixture of step (i) to an excitation light source capable of exciting the first and second QD populations,
wherein the relation between the photoluminescent intensities of the second and first QD populations is correlated with the quantity of hydrolytic enzyme in the sample.

14. The method according to claim 13, wherein the nanoparticle comprises a peptide comprising a site susceptible of being cleaved by trypsin and the sample is human faeces.

15. The method according to claim 14 that further comprises the step of diagnosing cystic fibrosis when the faecal trypsin concentration in the sample is comprised from 0 to 90 µg/g faeces.

16. A method for preparing a nanoparticle as defined in any of the claims 1-9 comprising:
(i) forming a core comprising a first population of silica embedded QDs by the reverse microemulsion method,
(ii) forming a shell containing a second population of silica embedded QDs by subjecting the core comprising a first population of silica embedded QDs of step (i) to reverse microemulsion method,
(iii) recovering the core-shell nanoparticle obtained in step (ii), and optionally functionalising the surface of the shell, and
(iv) adding at least one biomolecule selected from a peptide, a nucleic acid, a carbohydrate or a lipid, wherein each biomolecule comprises a cleavage site that is susceptible of being cleaved by a hydrolytic enzyme, and a photoluminescent label that is bound to the part of the biomolecule which detaches from the nanoparticle after cleavage of said biomolecule by a hydrolytic enzyme through a diradical of formula FG₃ as defined in claim 1; whereby the biomolecule is bound to the surface of the shell through a moiety selected from the moieties of formula (I) and (II) as defined in claim 1.

## Patentansprüche

1. Eine Nanopartikel umfassend
(i) einen Kern umfassend eine erste Gesamtheit von in Siliciumdioxid eingebetteten Quantenpunkten (QP),
(ii) eine Hülse umfassend eine zweite Gesamtheit von in Siliciumdioxid eingebetteten QP,
(iii) mindestens ein Biomolekül ausgewählt aus einem Peptid, einer Nukleinsäure, einem Kohlenhydrat oder einem Lipid, welches eine Spaltstelle umfasst, die mittels eines hydrolytischen Enzyms gespaltet werden kann, wobei das Biomolekül an der Oberfläche der Hülse durch einen Rest gebunden ist, der aus den Resten der Formel (I) und (II) ausgewählt ist, 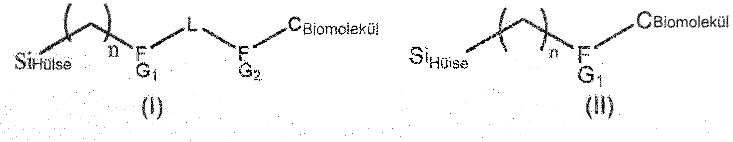
wobei Si_{Hülse} ein in der Hülse vorhandenes Siliciumatom ist und C_{Biomolekül} ein in dem Biomolekül vorhandenes Kohlenstoffatom ist;
n eine Ganzzahl von 0 bis 10 ist;
FG₁ und FG₂ jeweils Diradikale sind, die unabhängig voneinander aus der Gruppe bestehend aus den Radikalen der Formel (III), (IV), (V), (VI), (VII), (VIII) und (IX) ausgewählt sind 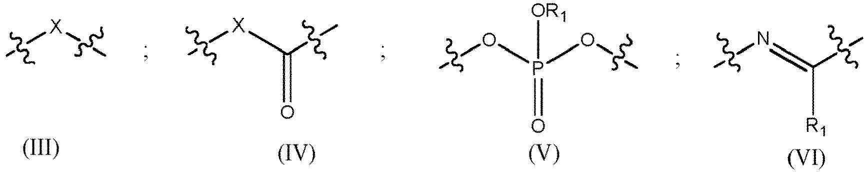 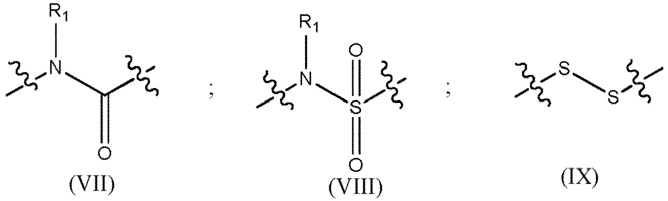
worin X = S oder O; R₁ = H oder (C₁-C₁₂)-Alkyl;
L ein Diradikal ist, das ausgewählt aus den Diradikalen ist, die aus einer Verbindung ausgewählt aus (C₁-C₁₂)-Alkan, (C₁-C₁₂)-Alken, (C₁-C₁₂)-Alkin, (C₃- C₆)-Cycloalkan und Benzol abgeleitet sind;
und
(iv) einen photolumineszenten Marker für jedes Biomolekül, wobei der Marker an dem Teil des Biomoleküls gebunden ist, welcher sich nach der Spaltung des Biomoleküls mittels des hydrolytischen Enzyms durch ein Diradikal der Formel FG₃, welches ausgewählt aus der Gruppe bestehend aus den Diradikalen der oben definierten Formel (III), (IV), (V), (VI), (VII), (VIII) und (IX) ist, von der Nanopartikel trennt;
wobei die ersten und zweiten QP-Gesamtheiten verschiedene maximale Photolumineszenz-Emissionswellenlängen haben, und nur die zweite QP-Gesamtheit Förster-Resonanzenergietransfer (FRET) mit dem photolumineszenten Marker ergeben kann.

2. Die Nanopartikel des Anspruchs 1, wobei das Biomolekül an der Oberfläche der Hülse durch einen Rest der Formel (I) gebunden ist, wobei:
n eine Ganzzahl von 1 bis 5 ist;
X = O; und
FG₁ und FG₂ unabhängig voneinander aus den Diradikalen der Formel (IV), (VI) und (VII) ausgewählt werden.

3. Die Nanopartikel nach einem der Ansprüche 1 bis 2, wobei FG₃ ausgewählt aus den Diradikalen der Formel (IV), (VII) und (VIII) ist.

4. Die Nanopartikel nach einem der Ansprüche 1 bis 3, wobei das Biomolekül ein Peptid umfassend eine Spaltstelle ist, die mittels eines proteolytischen Enzyms gespaltet werden kann, wobei die Sequenz des Peptids von 3 bis 12 Aminosäuren umfasst.

5. Die Nanopartikel nach einem der Ansprüche 1 bis 4, wobei das Biomolekül ein Peptid umfassend eine Spaltstelle ist, die von Trypsin gespaltet werden kann.

6. Die Nanopartikel nach einem der Ansprüche 1-5, wobei
(i) der durchschnittliche Abstand von dem photolumineszenten Marker zu der zweiten QP-Gesamtheit von 7 bis 22 nm reicht,
(ii) das photolumineszente Emissionsspektrum der zweiten QP-Gesamtheit und das photolumineszente Absorptionsspektrum des Markers eine spektrale Überlappung von 40 bis 80 % (Oberfläche/Oberfläche) haben; oder das photolumineszente Emissionsspektrum des Markers und das photolumineszente Absorptionsspektrum der zweiten QP-Gesamtheit eine spektrale Überlappung von 40 bis 80 % (Oberfläche/Oberfläche) haben,
(iii) der durchschnittliche Abstand von dem Marker zur der ersten QP-Gesamtheit von 25 bis 100 nm reicht und/oder das photolumineszente Emissionsspektrum der ersten QP-Gesamtheit und das photolumineszente Absorptionsspektrum des Markers eine spektrale Überlappung von 0 bis 30% (Oberfläche/Oberfläche) haben; oder der durchschnittliche Abstand von dem Marker zur der ersten QP-Gesamtheit von 25 bis 100 nm reicht und/oder das photolumineszente Emissionsspektrum des Markers und das photolumineszente Absorptionsspektrum der ersten QP-Gesamtheit eine spektrale Überlappung von 0 bis 30% (Oberfläche/Oberfläche) haben, und
(iv) der durchschnittliche Abstand zwischen der ersten QP-Gesamtheit und der zweiten QP-Gesamtheit von 25 bis 50 nm reicht und/oder das photolumineszente Emissionsspektrum der ersten QP-Gesamtheit und das photolumineszente Absorptionsspektrum der zweiten QP-Gesamtheit eine spektrale Überlappung von 0 bis 30% (Oberfläche/Oberfläche) hat; oder der durchschnittliche Abstand zwischen der ersten QP-Gesamtheit und der zweiten QP-Gesamtheit von 25 bis 50 nm reicht und/oder das photolumineszente Emissionsspektrum der zweiten QP-Gesamtheit und das photolumineszente Absorptionsspektrum der ersten QP-Gesamtheit eine spektrale Überlappung von 0 bis 30% (Oberfläche/Oberfläche) haben.

7. Die Nanopartikel nach Anspruch 6, wobei
(i) die erste QP-Gesamtheit eine maximale Photolumineszenz-Emissionswellenlänge von 600 bis 700 nm hat,
(ii) die zweite QP-Gesamtheit eine photolumineszente maximale Emissionswellenlänge von 500 bis 600 nm hat, und
(iii) der Marker eine photolumineszente maximale Absorptionswellenlänge von 450 bis 650 nm hat.

8. Die Nanopartikel nach einem der Ansprüche 1 bis 7, welche eine Struktur der Formel (X) gebunden an der Oberfläche der Hülse umfasst 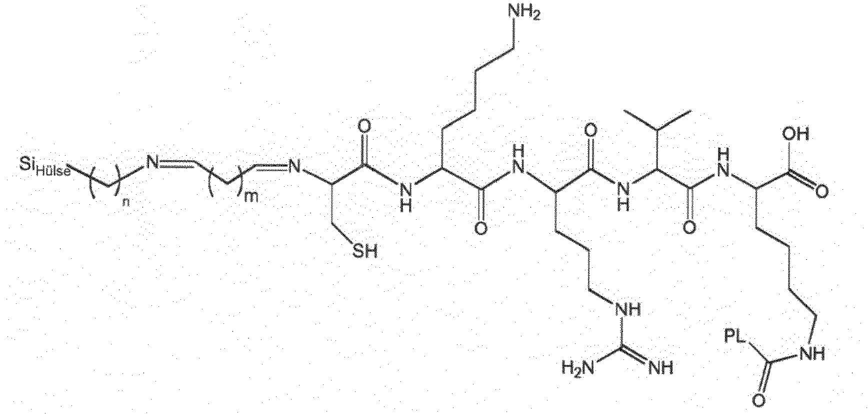
wobei m eine Ganzzahl von 1 bis 6 ist; und
PL ein photolumineszenter Marker ausgewählt aus Rhodamin oder einem Rhodaminderivat ist.

9. Die Nanopartikel nach einem der Ansprüche 1-8, wobei die QP ausgewählt aus der Gruppe bestehend aus CdSe, CdS; mit ZnS beschichtetem CdSe (CdSe/ZnS) und mit ZnS beschichtetem CdS (CdS/ZnS) sind.

10. Verwendung von einer Nanopartikel wie in einem der Ansprüche 1-9 definiert, zur Quantifizierung eines hydrolytischen Enzyms in einer Probe.

11. Verwendung nach Anspruch 10, wobei die Nanopartikel ein Peptid umfassend eine Spaltstelle umfasst, die von Trypsin gespaltet werden kann.

12. Verwendung von einer Nanopartikel wie in einem der Ansprüche 1-9 definiert als Diagnostikum der Mukoviszidose, wobei die Nanopartikel zur Quantifizierung eines hydrolytischen Enzyms in einer Probe verwendet wird, und sie ein Peptid umfassend eine Spaltstelle umfasst, die von Trypsin gespaltet werden kann.

13. Ein Verfahren zur Quantifizierung von einem hydrolytischen Enzym in einer Probe umfassend die folgenden Schritte:
(i) das in-Kontakt-Bringen einer Nanopartikel nach einem der Ansprüche 1-9 mit der Probe,
(ii) das Ermitteln der photolumineszenten Intensität der ersten und der zweiten QP-Gesamtheiten nach Aussetzung der Mischung aus dem Schritt (i) einer Anregungslichtquelle, welche die ersten und zweiten QP-Gesamtheiten anregen kann,
wobei das Verhältnis von photolumineszenten Intensitäten der zweiten und ersten QP-Gesamtheiten mit der Menge an hydrolytischem Enzym in der Probe korreliert.

14. Das Verfahren nach Anspruch 13, wobei die Nanopartikel ein Peptid umfassend eine Stelle umfasst, die von Trypsin gespaltet werden kann und die Probe eine Probe menschlichen Stuhls ist.

15. Das Verfahren nach Anspruch 14, welches weiterhin den Schritt umfasst, in dem die Mukoviszidose diagnostiziert wird, wenn die Trypsinkonzentration in der Stuhlprobe von 0 bis 90 µg/g Stuhl reicht.

16. Ein Verfahren zur Herstellung einer Nanopartikel wie in einem der Ansprüche 1-9 definiert umfassend:
(i) das Bilden eines Kerns umfassend eine erste Gesamtheit von in Siliciumdioxid eingebetteten QP durch das Umkehrmikroemulsionsverfahren,
(ii) das Bilden einer Hülse umfassend eine zweite Gesamtheit von in Siliciumdioxid eingebetteten QP, indem der Kern umfassend eine erste Gesamtheit von in Siliciumdioxid eingebetteten QP des Schritts (i) dem Umkehrmikroemulsionsverfahren ausgesetzt wird,
(iii) das Zurückgewinnen der im Schritt (ii) erhaltenen Kern-Hülse-Nanopartikel, und wahlweise funktionalisieren der Oberfläche der Hülse, und
(iv) das Hinzugeben von mindestens einem Biomolekül ausgewählt aus einem Peptid, einer Nukleinsäure, einem Kohlenhydrat oder einem Lipid, wobei jedes Biomolekül eine Spaltstelle umfasst, die mittels eines hydrolytischen Enzyms gespaltet werden kann, und einen photolumineszenten Marker umfasst, der an dem Teil des Biomoleküls gebunden ist, der sich nach der Spaltung des Biomoleküls mittels eines hydrolytischen Enzyms durch ein Diradikal der Formel FG₃ wie in Anspruch 1 definiert von der Nanopartikel trennt; wobei das Biomolekül an der Oberfläche der Hülse durch einen Rest gebunden ist, der ausgewählt aus Resten der Formel (I) und (II) wie in Anspruch 1 definiert gebunden ist.

## Revendications

1. Une nanoparticule comprenant
(i) un noyau comprenant un premier ensemble de points quantiques (PQ) intégrés dans de la silice
(ii) une gaine comprenant un deuxième ensemble de PQ intégrés dans de la silice
(iii) au moins une biomolécule choisie parmi un peptide, un acide nucléique, un carbohydrate ou un lipide qui comprend un site de clivage qui peut être clivé par un enzyme hydrolytique, étant ladite biomolécule liée à la surface de la gaine moyennant une fraction choisie parmi les fractions de formule (I) et (II), 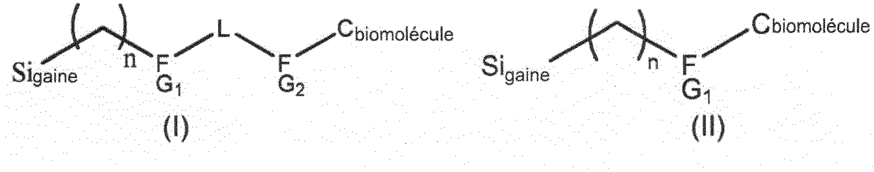
dans laquelle Si_{gaine} est un atome de silicium compris dans la gaine et C_{biomolécule} est un atome de carbone compris dans la biomolécule ;
n est un nombre entier allant de 0 à 10 ;
FG₁ et FG₂ sont chacun des diradicaux indépendamment choisis dans le groupe constitué des diradicaux de formule (III), (IV), (V), (VI), (VII), (VIII) et (IX) 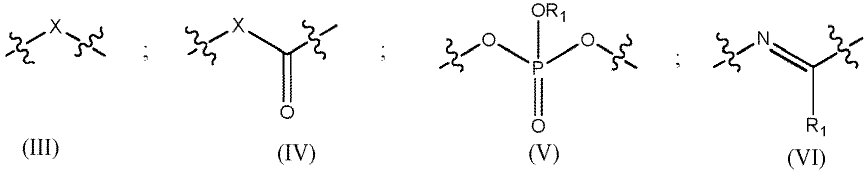 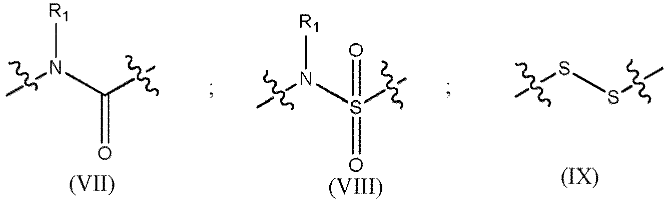
où X est S ou O ; R₁ est H ou alkyle en (C₁ à C₁₂) ;
L est un diradical choisi parmi les diradicaux dérivés d'un composé choisi parmi alcane en (C₁ à C₁₂), alcène en (C₁ en C₁₂), alcyne en (C₁ à C₁₂), cycloalcane en (C₃ à C₆) et benzène ;
et
(iv) un marqueur photoluminescent pour chaque biomolécule, où le marqueur est lié à la partie de la biomolécule qui se sépare de la nanoparticule après le clivage de ladite biomolécule par un enzyme hydrolytique par le biais d'un diradical de formule FG₃ qui est choisi dans le groupe constitué des diradicaux de formule (III), (IV), (V), (VI), (VII), (VIII) et (IX) définis ci-dessus ;
dans lequel les ensembles premier et deuxième de PQ ont une longueur d'onde d'émission de photoluminescence maximale différente, et seulement le deuxième ensemble de PQ peut produire du transfert d'énergie par résonance de type Förster (FRET) avec le marqueur photoluminescent.

2. La nanoparticule de la revendication 1, dans laquelle la biomolécule est liée à la surface de la gaine moyennant une fraction de formule (I) dans laquelle :
n est un nombre entier allant de 1 à 5 ;
X est O ; et
FG₁ et FG₂ sont indépendamment choisis parmi les diradicaux de formule (IV), (VI) et (VII).

3. La nanoparticule selon l'une quelconque des revendications 1 à 2, dans laquelle FG₃ est choisie parmi les diradicaux de formule (IV), (VII) et (VIII).

4. La nanoparticule selon l'une quelconque des revendications 1 à 3, dans laquelle la biomolécule est un peptide comprenant un site de clivage qui peut être clivé par une enzyme protéolytique, comprenant la séquence dudit peptide de 3 à 12 acides aminés.

5. La nanoparticule selon l'une quelconque des revendications 1 à 4, dans laquelle la biomolécule est un peptide comprenant un site de clivage qui peut être clivé par la trypsine.

6. La nanoparticule selon l'une quelconque des revendications 1-5, dans laquelle
(i) la distance moyenne entre le marqueur photoluminescent et le deuxième ensemble de PQ est comprise de 7 à 22 nm,
(ii) le spectre d'émission photoluminescent du deuxième ensemble de PQ et le spectre d'absorption photoluminescent du marqueur ont un chevauchement spectral allant de 40 à 80 % (surface / surface) ; ou le spectre d'émission photoluminescent du marqueur et le spectre d'absorption photoluminescent du deuxième ensemble de PQ ont un chevauchement spectral allant de 40 à 80 % (surface / surface),
(iii) la distance moyenne entre le marqueur et le premier ensemble de PQ va de 25 à 100 nm et/ou le spectre d'émission photoluminescent du premier ensemble de PQ et le spectre d'absorption photoluminescent du marqueur ont un chevauchement spectral allant de 0 à 30 % (surface / surface) ; ou la distance moyenne entre le marqueur et le premier ensemble de PQ va de 25 à 100 nm et/ou le spectre d'émission photoluminescent du marqueur et le spectre d'absorption photoluminescent du premier ensemble de PQ ont un chevauchement spectral allant de 0 à 30 % (surface / surface), et
(iv) la distance moyenne entre le premier ensemble de PQ et le deuxième ensemble de PQ va de 25 à 50 nm et/ou le spectre d'émission photoluminescent du premier ensemble de PQ et le spectre d'absorption photoluminescent du deuxième ensemble de PQ ont un chevauchement spectral de 0 à 30 % (surface / surface) ; ou la distance moyenne entre le premier ensemble de PQ et le deuxième ensemble de PQ va de 25 à 50 nm et/ou le spectre d'émission photoluminescent du deuxième ensemble de PQ et le spectre d'absorption photoluminescent du premier ensemble de PQ ont un chevauchement spectral de 0 à 30 % (surface / surface).

7. La nanoparticule selon la revendication 6, dans laquelle
(i) le premier ensemble de PQ a une longueur d'onde d'émission maximale photoluminescente allant de 600 à 700 nm,
(ii) le deuxième ensemble de PQ a une longueur d'onde d'émission maximale photoluminescente allant de 500 à 600 nm, et
(iii) le marqueur a une longueur d'onde d'absorption maximale photoluminescente allant de 450 à 650 nm.

8. La nanoparticule selon l'une quelconque des revendications 1 à 7, qui comprend une structure de formule (X) liée à la surface de la gaine 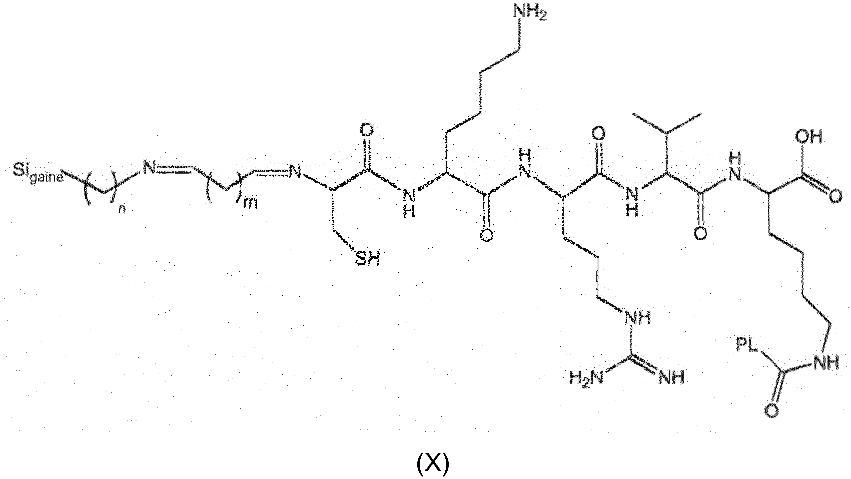
où m est un nombre entier de 1 à 6 ; et
PL est un marqueur photoluminescent choisi de la rhodamine ou d'un dérivé de la rhodamine.

9. La nanoparticule selon l'une quelconque des revendications 1-8, dans laquelle les PQ sont choisis dans le groupe constitué du CdSe, du CdS, du CdSe recouvert avec du ZnS (CdSe / ZnS) et du CdS recouvert avec du ZnS (CdS / ZnS).

10. Utilisation d'une nanoparticule telle que définie dans l'une quelconque des revendications 1-9, pour quantifier une enzyme hydrolytique dans un échantillon.

11. Utilisation selon la revendication 10, dans laquelle la nanoparticule comprend un peptide comprenant un site de clivage qui peut être clivé par la trypsine.

12. Utilisation d'une nanoparticule telle que définie dans l'une quelconque des revendications 1-9, comme agent diagnostique de fibrose kystique, dans laquelle la nanoparticule est utilisée pour quantifier une enzyme hydrolytique dans un échantillon, et elle comprend un peptide comprenant un site de clivage qui peut être clivé par la trypsine.

13. Un procédé pour quantifier une enzyme hydrolytique dans un échantillon comprenant les étapes suivantes :
(i) contacter une nanoparticule selon l'une quelconque des revendications 1-9 avec l'échantillon,
(ii) déterminer l'intensité photoluminescente du premier et du deuxième ensemble de PQ après l'exposition du mélange de l'étape (i) à une source de lumière d'excitation capable d'exciter les ensembles de PQ premier et deuxième,
dans lequel la relation entre les intensités photoluminescentes des ensembles de PQ deuxième et premier sont corrélées à la quantité d'enzyme hydrolytique dans l'échantillon.

14. Le procédé selon la revendication 13, dans lequel la nanoparticule comprend un peptide comprenant un site qui peut être clivé par la trypsine et l'échantillon est un échantillon de fèces humaines.

15. Le procédé selon la revendication 14 qui comprend en outre l'étape de diagnostiquer de la fibrose kystique lorsque la concentration fécale de trypsine dans l'échantillon va de 0 à 90 µg/g fèces.

16. Un procédé de préparation d'une nanoparticule telle que définie dans l'une quelconque des revendications 1-9 comprenant :
(i) former un noyau comprenant un premier ensemble de PQ intégrés dans de la silice moyennant le procédé de microémulsion inverse,
(ii) former une gaine contenant un deuxième ensemble de PQ intégrés dans de la silice en soumettant le noyau comprenant un premier ensemble de PQ intégrés dans de la silice de l'étape (i) à la méthode de microémulsion inverse,
(iii) récupérer la nanoparticule noyau-gaine obtenue dans l'étape (ii), et fonctionnaliser de façon facultative la surface de la gaine, et
(iv) ajouter au moins une biomolécule choisie parmi un peptide, un acide nucléique, un carbohydrate ou un lipide, dans lequel chaque biomolécule comprend un site de clivage qui peut être clivé par une enzyme hydrolytique, et un marqueur photoluminescent qui est lié à la partie de la biomolécule qui se sépare de la nanoparticule après le clivage de ladite biomolécule par une enzyme hydrolytique par le biais d'un diradical de formule FG3 tel que défini dans la revendication 1 ; où la biomolécule est liée à la surface de la gaine par voie d'une fraction choisie parmi les fractions de formule (I) et (II) telles que définies dans la revendication 1.


### SEQUENCE LISTING

<110> Fundació Institut Català d'Investigació Química
<120> Ratiometric assay for hydrolytic enzyme quantification
<130> P2648PC00
<150> EP13382239.5
   <151> 2013-06-25
<160> 3
<170> PatentIn version 3.5
<210> 1
   <211> 5
   <212> PRT
   <213> Artificial Sequence
<220>
   <223> Pro-active peptide
<400> 1
   Cys Lys Arg Val Lys
   1 5
<210> 2
   <211> 5
   <212> PRT
   <213> Artificial Sequence
<220>
   <223> Pro-active peptide, TAMRA labelled
<220>
   <221> MOD_RES
   <222> (5)..(5)
   <223> TAMRA-labelled aminoacid
<400> 2
   Cys Lys Arg Val Lys
   1 5
<210> 3
   <211> 5
   <212> PRT
   <213> Artificial Sequence
<220>
   <223> in-active peptide, TAMRA labelled
<220>
   <221> MOD_RES
   <222> (5)..(5)
   <223> TAMRA-labelled aminoacid
<400> 3
   Cys Lys Pro Val Lys
   1 5